# EUROPEAN PATENT SPECIFICATION

(11) **EP 0 183 445 B1**
(45) Date of publication and mention of the grant of the patent: **29.08.1990**
(21) Application number: 85308303.8
(22) Date of filing: 14.11.1985
(51) Int. Cl.: G03C 7/38, C07D 487/04

(54) **Silver halide color photo-sensitive material**
Farbphotoempfindliches Silberhalogenidmaterial
Matériau photosensible couleur à l'halogénure d'argent

(30) Priority: 15.11.1984 JP 243008/84; 14.12.1984 JP 264139/84
(43) Date of publication of application: 04.06.1986
(73) Proprietor: KONICA CORPORATION, Tokyo 163 (JP)
(72) Inventor: Nakayama, Noritaka, Hino-shi Tokyo (JP); Kawakatsu, Satoshi, Hino-shi Tokyo (JP); Katoh, Katsunori, Hino-shi Tokyo (JP); Shinozaki, Kaoru, Hino-shi Tokyo (JP)
(74) Representative: Ellis-Jones, Patrick George Armine

(56) References cited:
- EP-A- 0 177 765
- EP-A- 0 178 789
- DE-A- 1 810 464

## Description

### Background of the Invention

### Field of the Invention

The present invention relates to a silver halide color photographic material that contains a magenta coupler capable of effective color formation and which forms a magneta dye image having improved keeping qualities, particularly in terms of light fastness. More specifically, the invention relates to a silver halide color photographic material containing a novel class of 1H-pyrazolo[3,2-c]-S-triazole derived magenta couplers.

### Description of the Prior Art

The formation of dye images in most silver halide color photographic materials depends on the reduction of exposed silver halide grains with an aromatic primary amine color developing agent and the subsequent coupling of the resultant oxidation product of the color developing agent with couplers that respectively form yellow, magenta and cyan dyes.

Pyrazolone type couplers are commercially used as couplers for providing magenta dyes, but they have an unwanted secondary absorption and their keeping qualities, particularly their resistance to formalin (formaldehyde) gas, is relatively low.

A variety of 1 H-pyrazolo[3,2-c]-S-triazole derived magenta couplers have been proposed to overcome these problems of the conventional pyrazolone type couplers. Reference should be made to U.S. Patent No. 3,725,067, as well as British Patent Nos. 1,252,418 and 1,334,515. The compounds disclosed in these patents avoid the problem of secondary absorption but the improvement is inadequate in terms of resistance to formalin gas and is insignificant in respect of the production of a light-fast magenta dye image. The compound disclosed in Research Disclosure No. 12443 has no commercial value because of its low color formation. The 1H-pyrazoio[3,2-c]-S-triazoie type magenta coupler disclosed in Unexamined Published Japanese Patent Application No. 42045/1983 features significant improvements in formalin resistance and color formation but little improvement has been achieved in terms of the production of a light-fast image.

Improved color development has also been achieved by the couplers described in Unexamined Published Japanese Patent Application Nos. 99437/1984 and 125732/1984 but the dye images produced by these couplers still possess poor light fastness. The coupler disclosed in Unexamined Published Japanese Patent Application No. 99437/1984 depends on the concomitant use of additives for providing a light-fast image. The coupler disclosed as Compound No. 19 in Unexamined Published Japanese Patent Application No. 125732/1984 produces a dye image having slightly improved light fastness but the improvement is far from being satisfactory.

DE-A-1810464 discloses a photographic material which comprises a silver halide emulsion layer containing a magenta 1H-pyrazolo[3,2-c]-S-triazole coupler in which the 7 position can be substituted by, for instance, chlorine and the 3 and/or 6 position can be substituted, for example by alkyl groups including isopropyl and secondary butyl.

EP-A-0177765, which may form part of the art under Article 54(3) EPC, discloses a photographic material which comprises a silver halide emulsion layer containing a magenta coupler which may be a 1H-pyrazolo[3,2-c]-S-triazole coupler. There is no specific mention of a secondary alkyl substituent in the 6-position although two such compounds are listed. These compounds are identified in the disclaimer in claim 1 of this application.

In short, the 1 H-pyrazolo[3,2-c]-S-triazole derived magenta couplers that have been considered useful because of the absence of secondary absorption and their high resistance to formalin gas fall far short of satisfying the requirement for providing dye images with improved light fastness.

### Summary Of The Invention

The primary object, therefore, of the present invention is to provide a silver halide color photographic material that contains a magenta coupler capable of effective color formation and which forms a magenta dye image having improved light fastness and resistance to formalin gas.

According to the present invention there is provided a silver halide color photographic material that has at least one silver halide emulsion layer on a support, said silver halide emulsion layer containing at least one 1H-pyrazoio[3,2-ci-S-triazoie type magenta coupler substituted at 6-position by an optionally substituted secondary alkyl group and at 7-position by a leaving group other than a hydrogen atom susceptible to displacement during reaction of the compound with the oxidation product of a color developing agent.

As a result of serious studies made to achieve the stated object, the inventors have found a 1H-pyrazoio[3,2-c]-S-triazote derived magenta coupler that exhibits effective color formation and which provides a magenta dye image having improved formalin resistance and light fastness.

That is to say, by the use of such a coupler, it was possible to improve light-fastness greatly and also to achieve effective color development.

As for the leaving group, the 7-position of a 1H-pyrazolo[3,2-c]-S-triazole type compound is a position at which to couple with the oxidation product of a color developing agent to form a magenta dye, at which time the group at 7-position leaves upon reaction with said oxidation product of a color developing agent.

A more preferable 1H-pyrazolo[3,2-c]-S-triazole type compound substituted at 6-position by a secondary alkyl group and at 7-position by a leaving group other than a hydrogen atom is represented by the following formula (I): 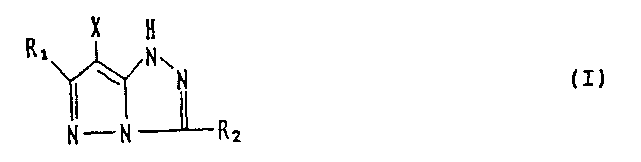 wherein R₁ is a secondary alkyl group; R₂ is an alkyl group, an aryl group, a heterocyclic group or a group bonded to the carbon atom of the nucleus through a bonding group selected from among an oxygen atom, a nitrogen atom, a sulfur atom, acylamino, carbamoyl, sulfonamido, sulfamoyl-carbonyl, carbonyloxy, oxycarbonyl, ureido, thioureido, thioamido, sulfone and sulfonyloxy groups; and

X is a leaving agent other than a hydrogen atom that leaves upon reaction with the oxidation product of a color developing agent.

The compound of formula (I) is preferably represented by the following formula (II): 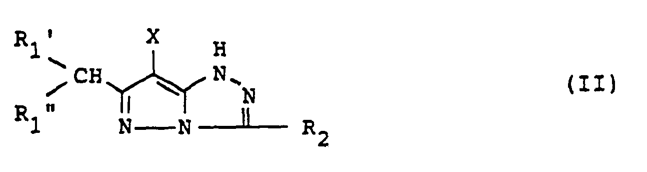 wherein R', and R", each idependently represent a halogen atom, a cyano group, a nitro group or a group which is in the same as one represented by R₂.in formula (I);
R₂ represents a group which is the same as one represented by R₂ in formula (I); and
X is the same in meaning as X in formula (I).
R', and R''₁ may be bonded to each other to complete a carbon ring or a heterocyclic ring.

The group represented by R'₁ and R''₁ is a halogen atom (e.g. chlorine, bromine or fluorine), or a straight- or branched-chain alkyl group having 1 to 20 carbon atoms (e.g. methyl, ethyl, propyl, i-propyl, sec-butyl, n-butyl, t-butyl, n-octyl, t-octyl, dodecyl or octadecyl). These groups may have a substituent(s) such as halogen atom, nitro, cyano, alkoxy, aryloxy, amino, acylamino, carbamoyl, sulfonamido, sulfamoyl, imido, alkylthio, arylthio, aryl, alkoxycarbonyl or acyl. Examples of such substituent include chloromethyl, bromomethyl, trichloromethyl, β-nitroethyl, b-cyanobutyl, methoxymethyl, ethoxyethyl, phenoxyethyl, N-methylaminoethyl, dimethylaminobutyl, acetoaminoethyl, benzoylamino, propyl, ethyl- carbamoylethyl, methanesulfonamidoethyl, ethylthioethyl, p-methoxyphenylthiomethyl, phenylmethyl, p-chlorophenylmethyl, naphthylethyl, ethoxycarbonylethyl and acetylethyl.

The aryl group includes phenyl and naphthyl groups and may have substituent(s) as shown with respect to the alkyl group.

The heterocyclic ring represents a 5- or 6-membered ring having at least one nitrogen, oxygen or sulfur atoms, and may be one which does or does not have aromaticity. Examples of such ring include pyridyl, quinolyl, pyrrolyl, morpholyl, furanyl, tetrahydrofuranyl, pyrazolyl, triazolyl, tetrazolyl, thiazolyl, oxazolyl, imidazolyl and thiadiazolyl. These may also have substituent(s) as shown with respect to the alkyl group.

R', and R", may also be bonded to each other to complete a carbon ring (e.g. cyclopropyl, cyclopentyl, cyclohexyl or cyclohexenyl) ora heterocyclic ring (e.g. piperidyl, pyrrolidyl, dioxanyl or morphorinyl).

Examples of R₁ in formulas (I) and (II) are shown below:

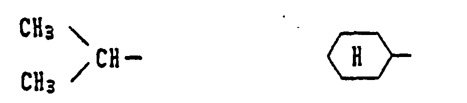

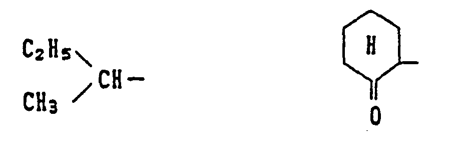

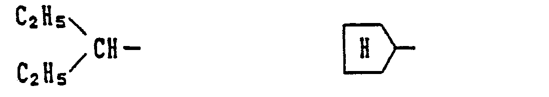

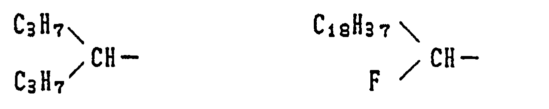

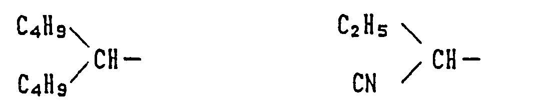

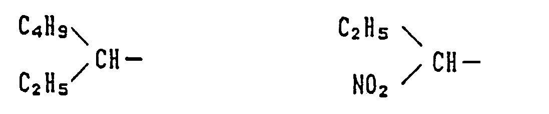

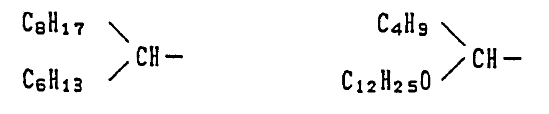

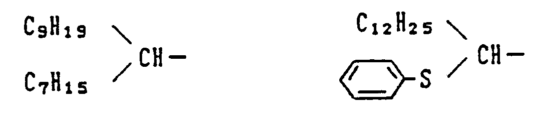

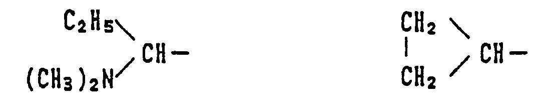

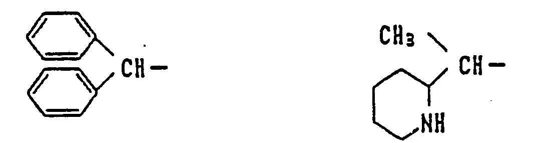

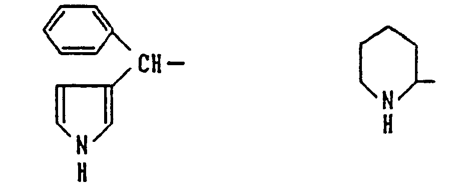

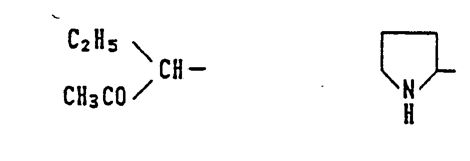

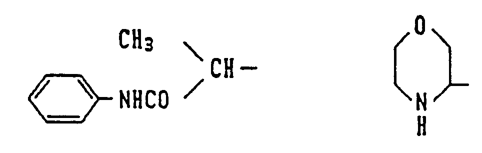

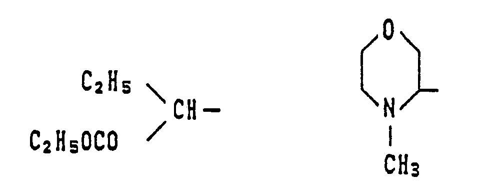

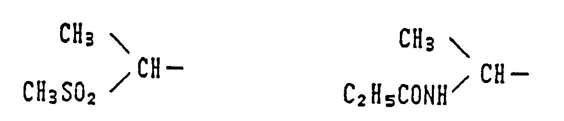

Examples of the alkyl, aryl or heterocyclic group represented by R₂ bonded through said bonding group or a nitrogen, oxygen or sulfur atom are shown below: 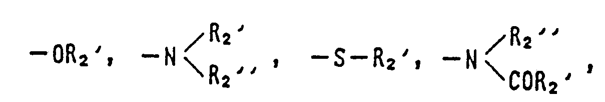 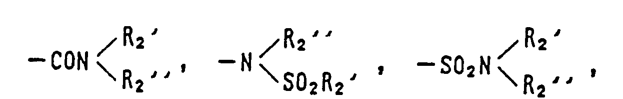 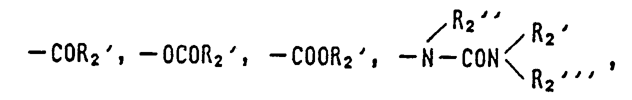 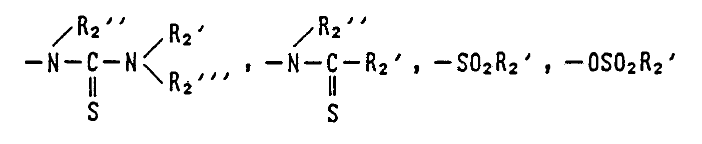 wherein R₂' represents an alkyl, aryl or heterocyclic group; and R₂" and R₂''' each represents a hydrogen atom, an alkyl group, an aryl group or a heterocyclic ring.

If the heterocyclic group is a 1 H-pyrazolo[3,2-c]-S-triazole-3-yl compound, a bis type 1 H-pyrazolo[3,2-c] -S-triazole compound is formed and this is of course a magenta coupler included within the scope of the present invention.

The leaving group represented by X is a halogen atom or an organic group bonded to the coupling site through an oxygen atom, a nitrogen atom or a sulfur atom.

Illustrative leaving groups that are bonded to the coupling site through an oxygen atom include alkoxy, aryloxy, acyloxy and heterocyclicoxy; exemplary leaving groups that are bonded to the coupling site through a nitrogen atom include acylamino, diacylamino, sulfonamido, and 5- or 6-membered heterocyclic groups containing that nitrogen atom; and illustrative leaving groups that are bonded to the coupling site through a sulfur atom include thiocyano, alkylthio, arylthio, heterocyclicthio, arylsulfonyl and alkylsulfonyl.

Examples of R₂ in formulas (I) and (II) are shown below:

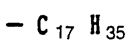

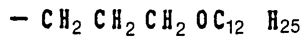

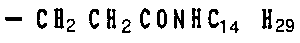

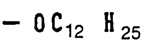

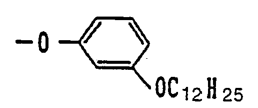

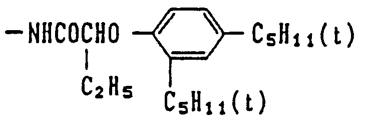

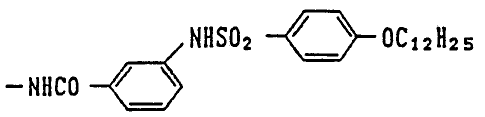

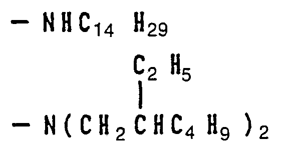

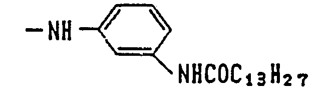

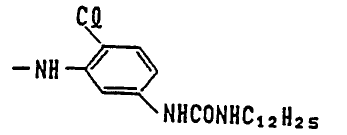

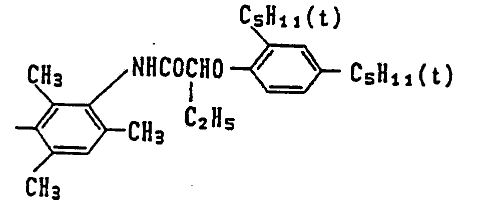

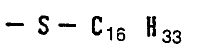

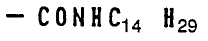

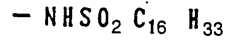

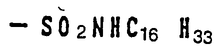

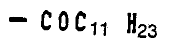

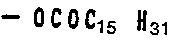

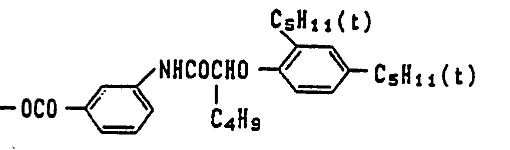

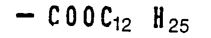

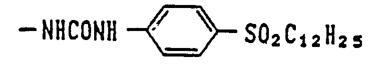

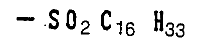

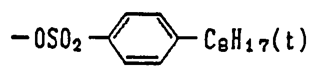

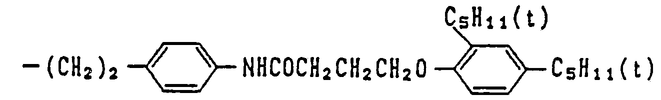

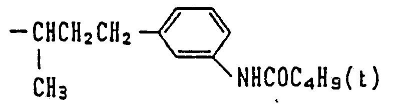

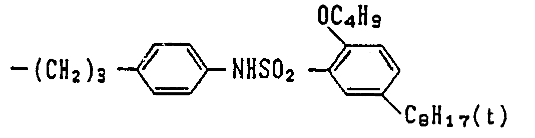

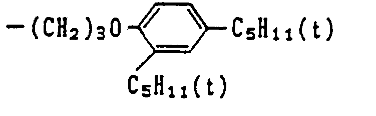

For R₁' and R₁'', preferred are alkyl and aryl groups and more preferably unsubstituted alkyl groups. Further, R,' and R₁' may be bonded to each other to complete a carbon ring.

Most preferable R₁ groups are shown below: 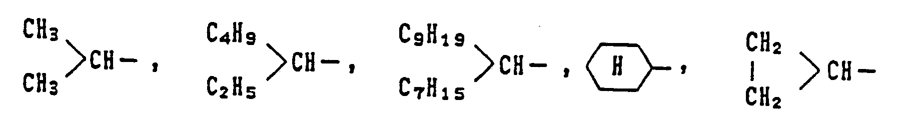
R₂ is preferably an alkyl or aryl group.
R₂ is more preferably a group represented by any of the following formulas (III), (IV) and (V): 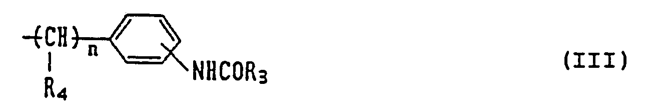 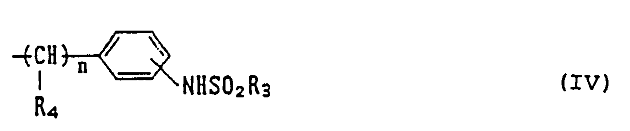 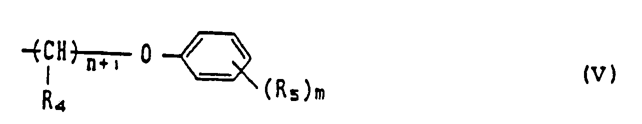 wherein n is an integer of 0 to 4; m is an integer of 1 to 3; R₃ is an alkyl, aryl or heterocyclic group; R₄ is a hydrogen atom or an alkyl group; and R₅ is an alkyl, alkoxy, sulfamoyl, sulfonamido or sulfonyl group.

The 1H-pyrazolo[3,2-c]-S-triazole derived magenta couplers in accordance with the present invention are illustrated by, but by no means limited to, the following compounds.

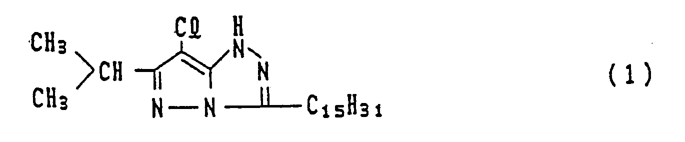

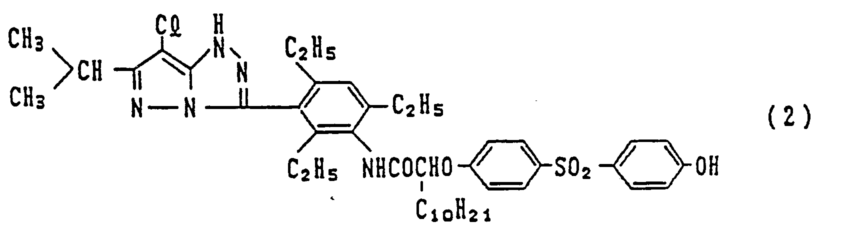

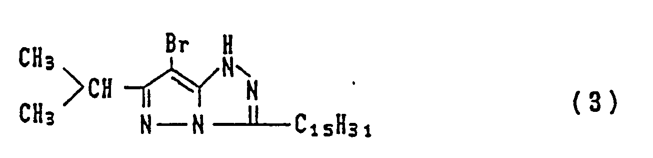

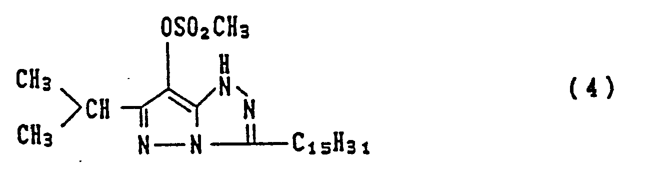

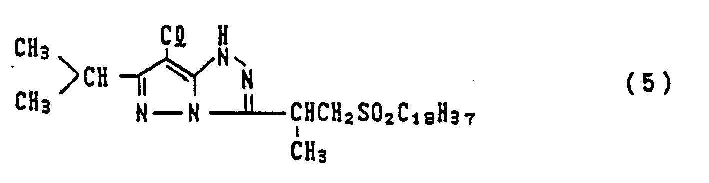

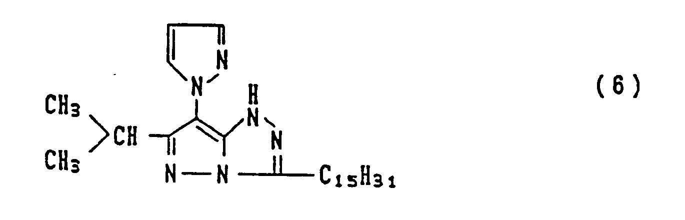

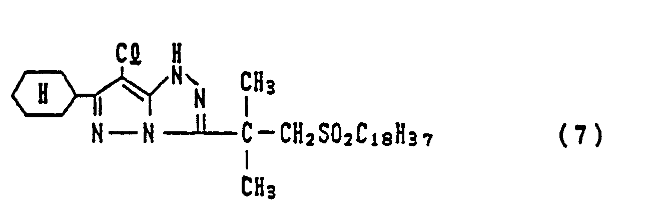

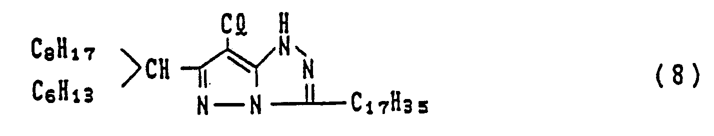

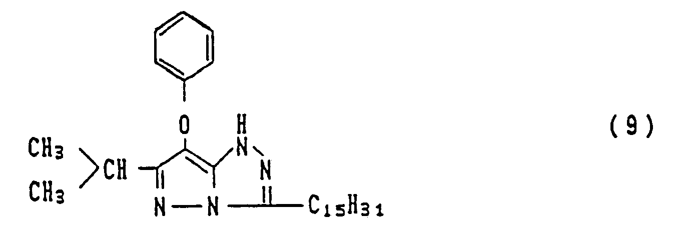

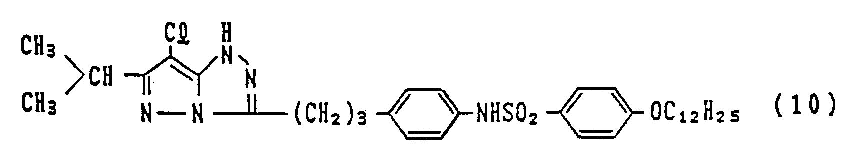

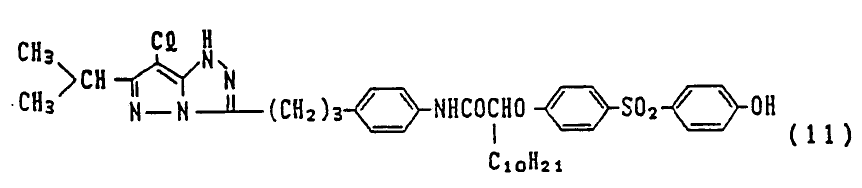

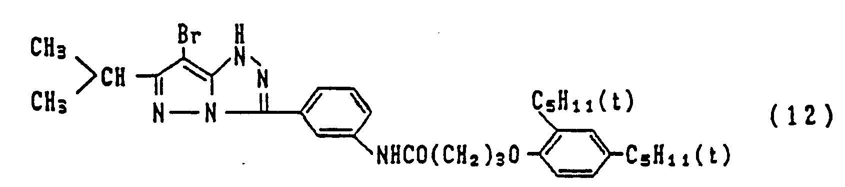

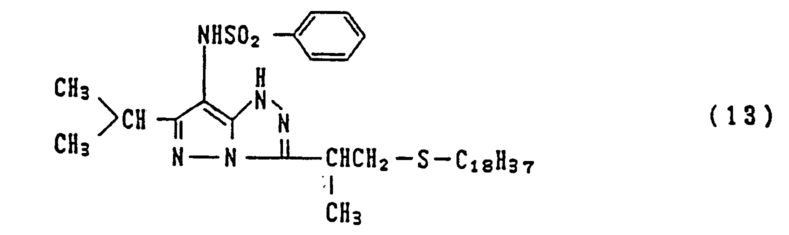

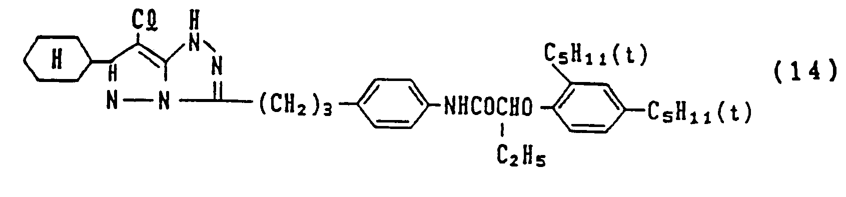

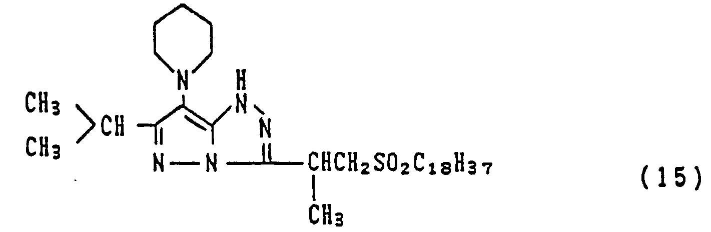

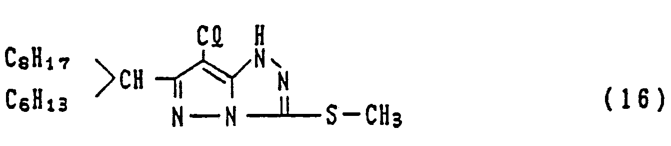

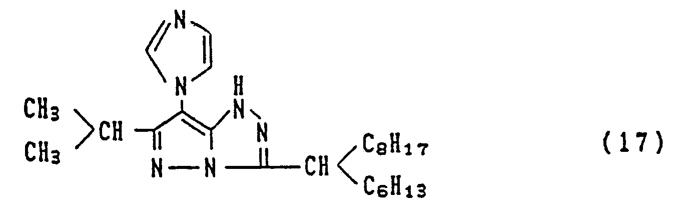

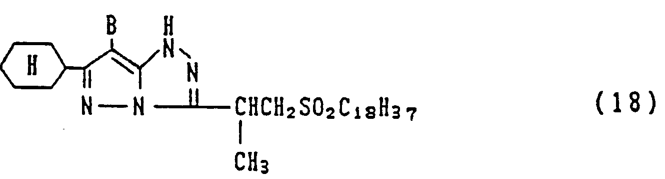

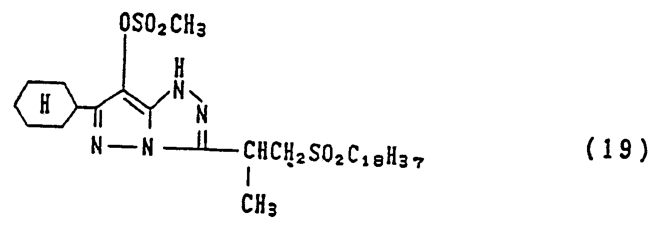

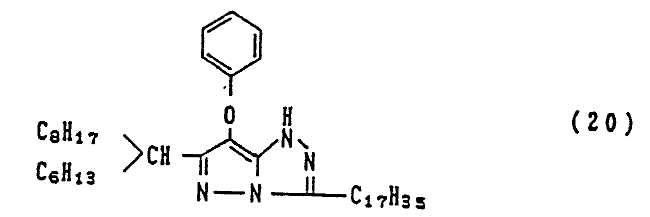

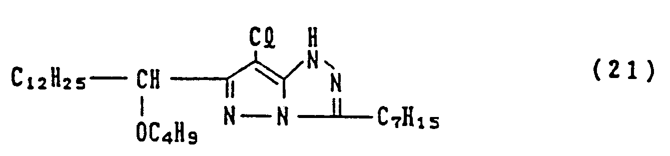

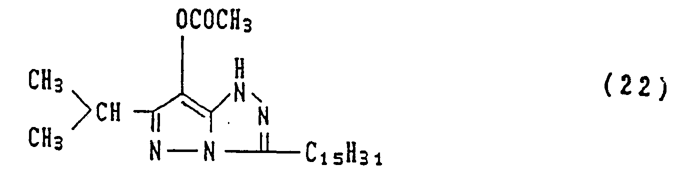

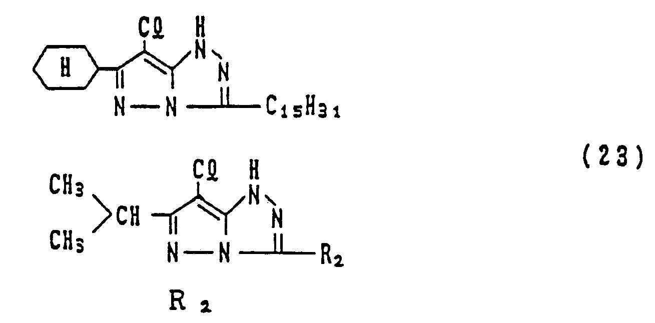

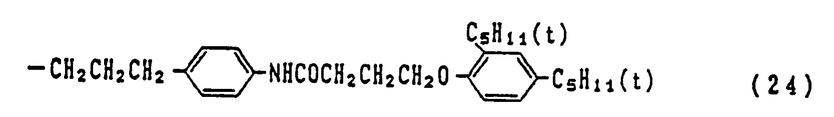

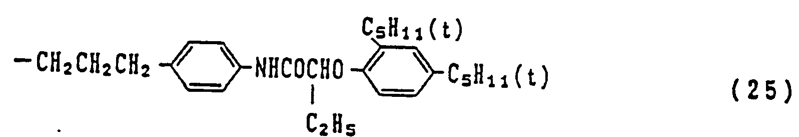

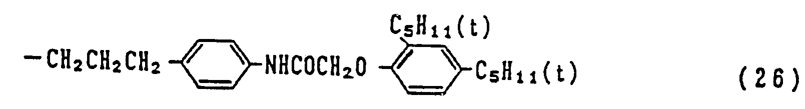

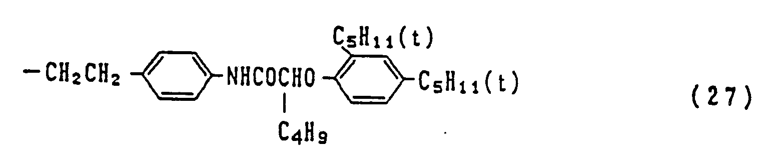

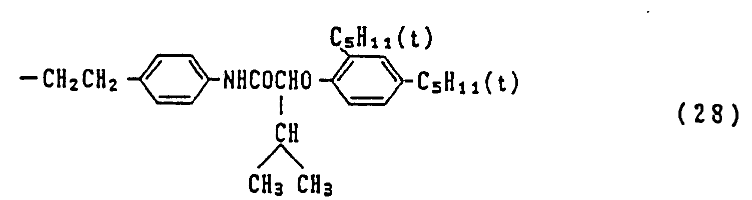

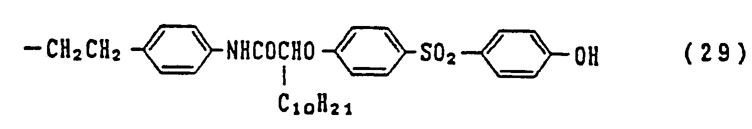

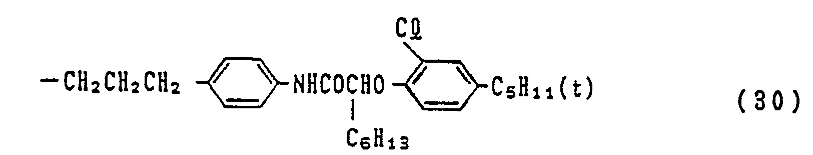

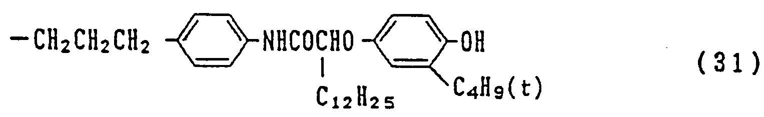

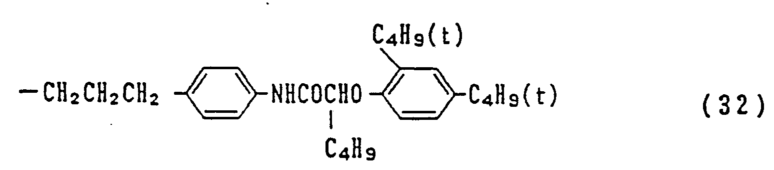

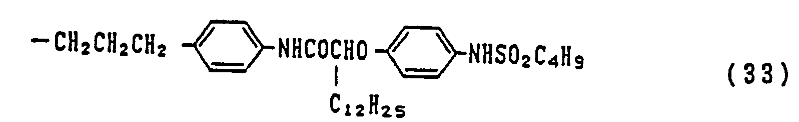

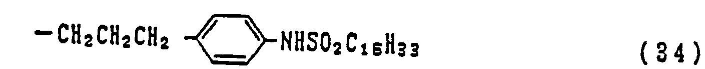

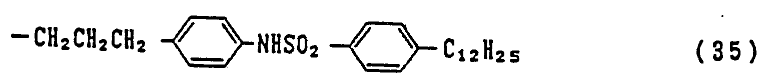

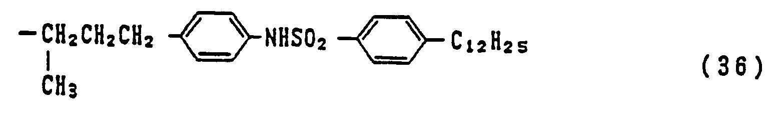

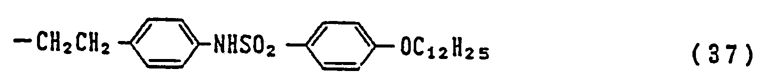

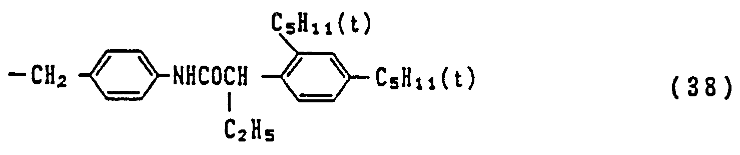

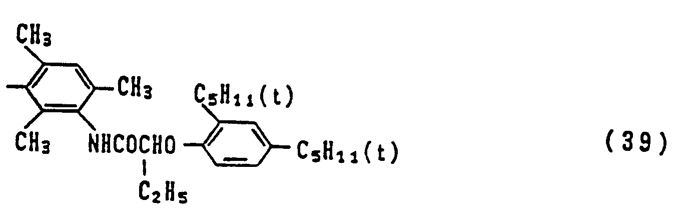

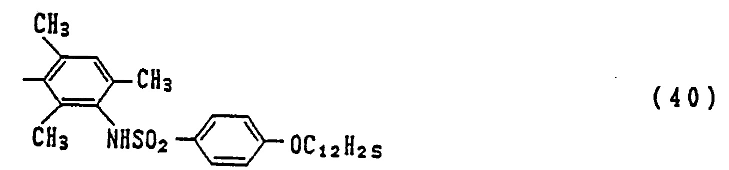

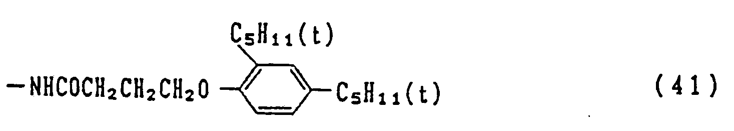

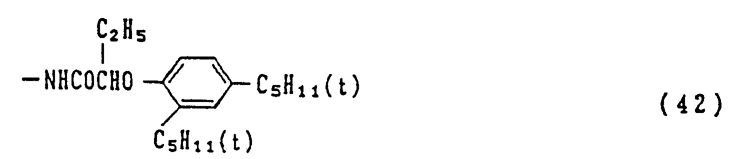

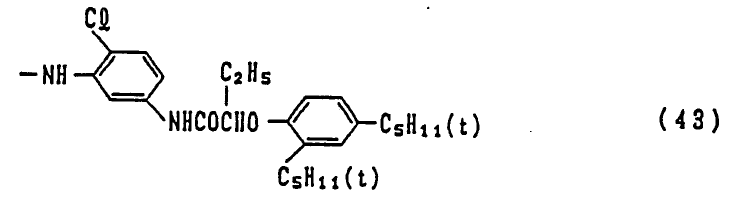

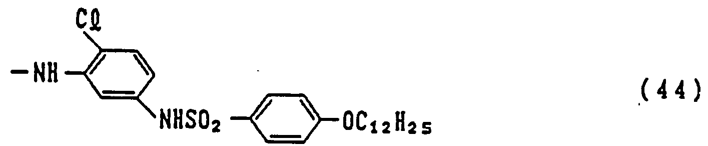

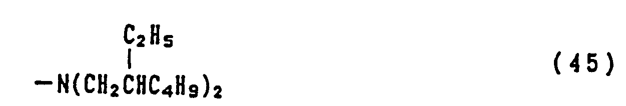

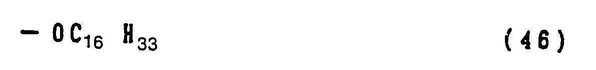

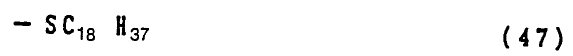

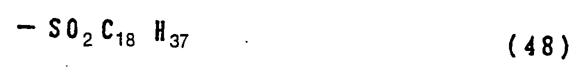

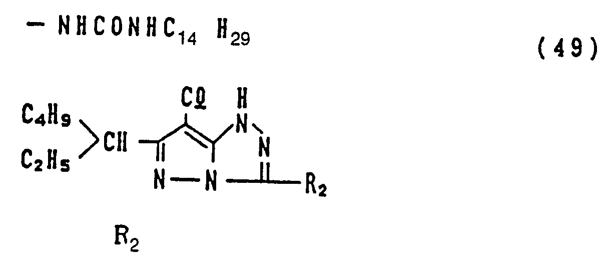

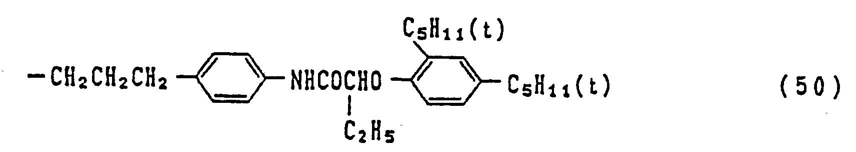

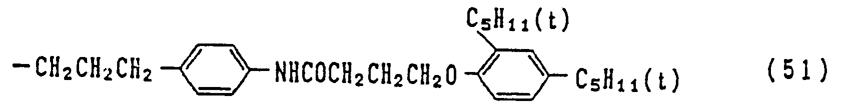

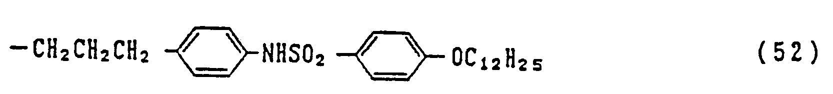

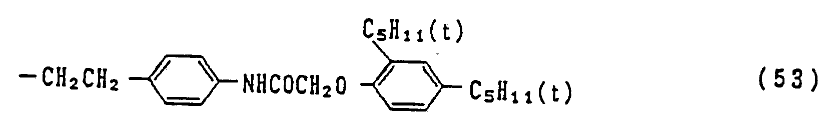

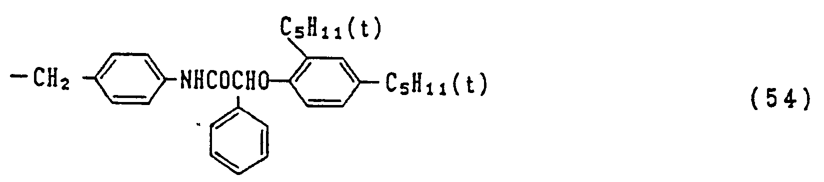

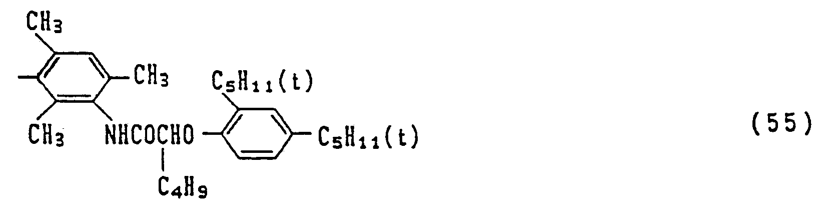

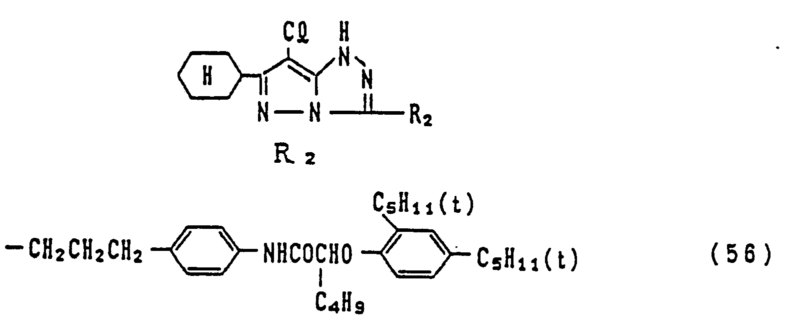

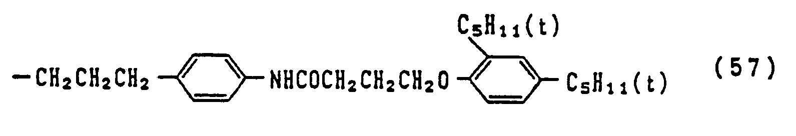

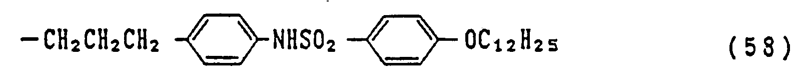

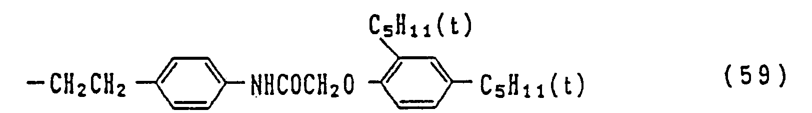

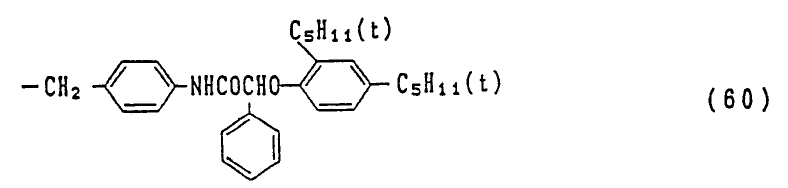

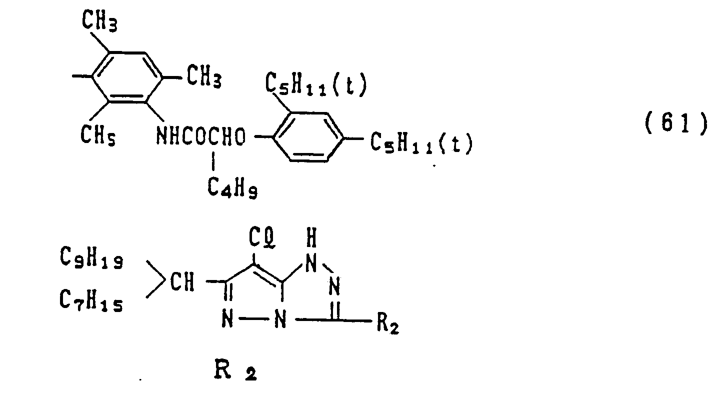

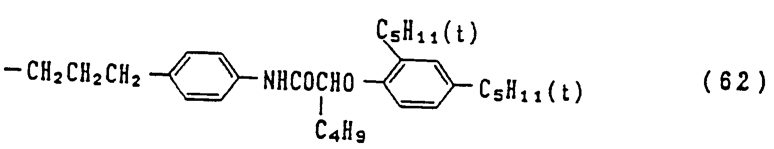

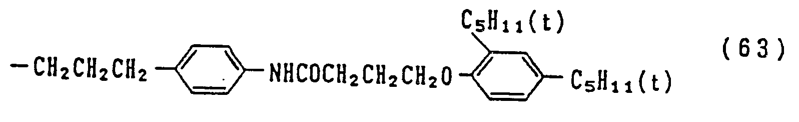

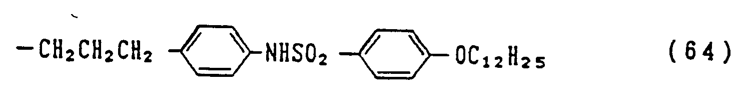

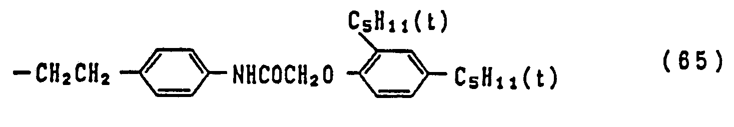

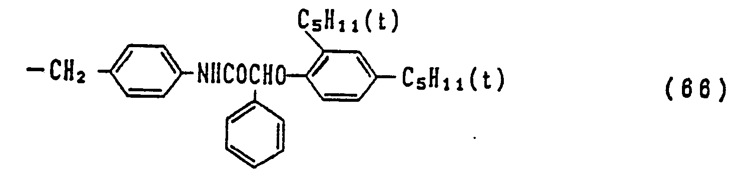

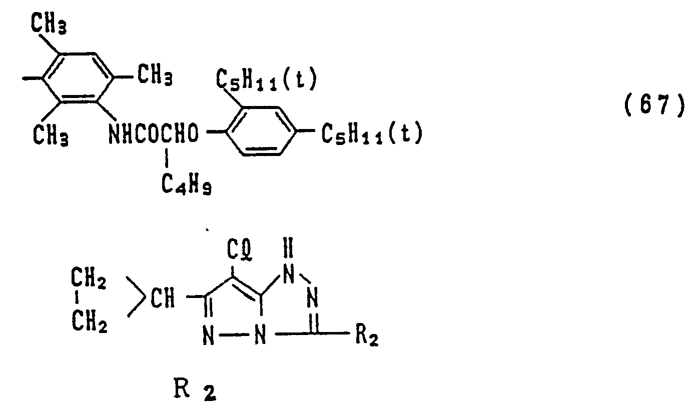

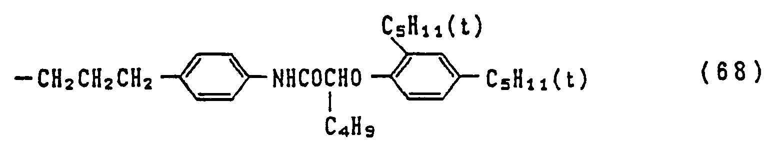

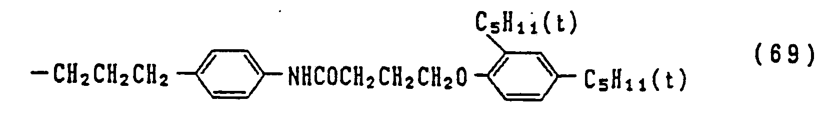

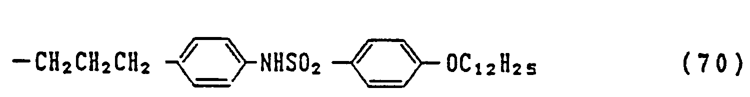

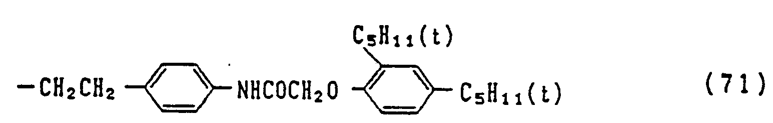

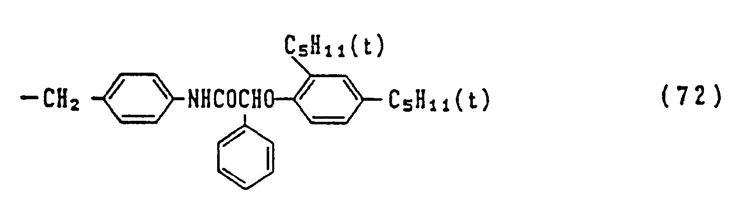

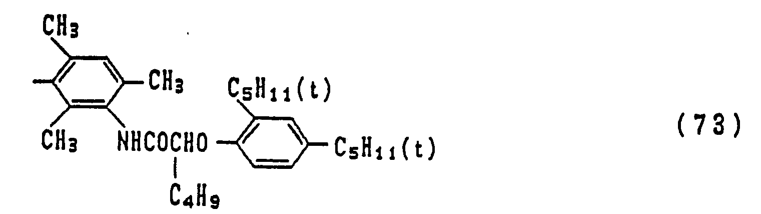

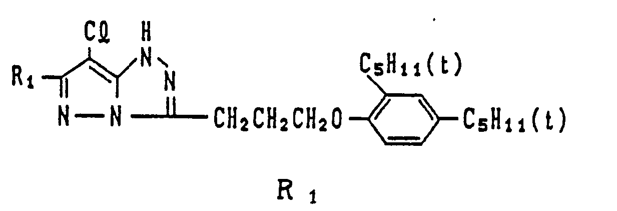

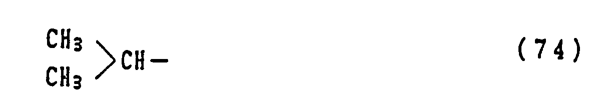

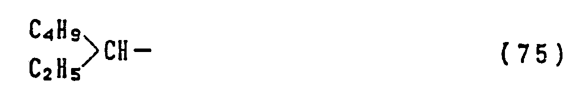

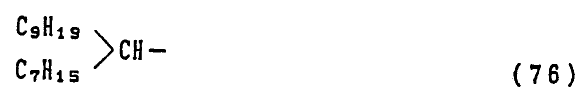

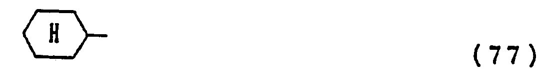

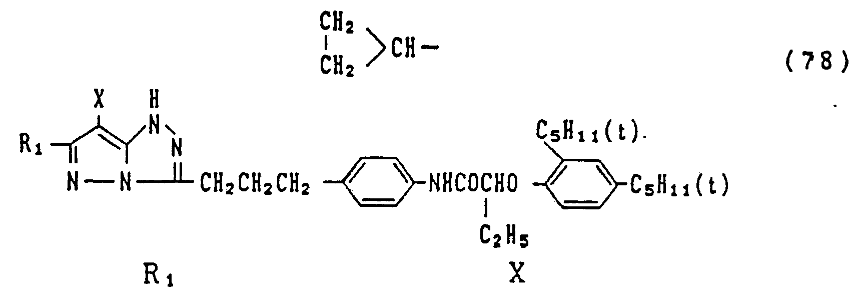

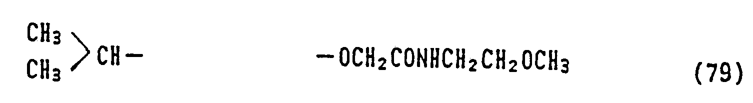

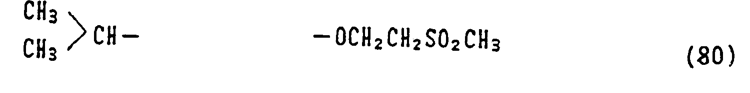

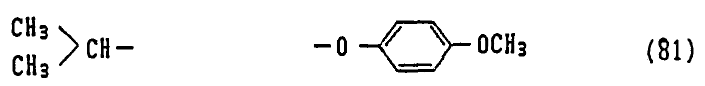

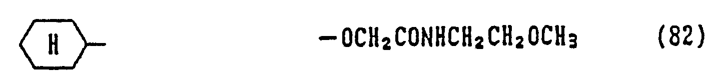

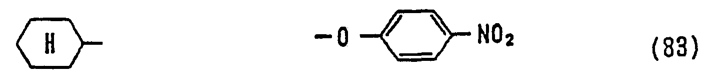

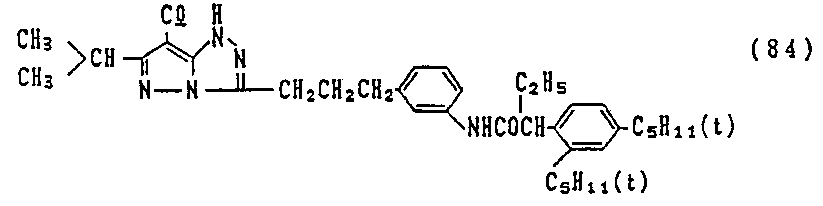

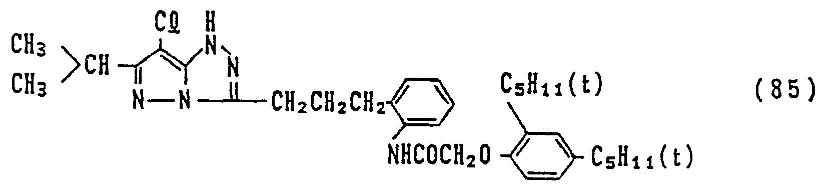

The methods for synthesizing several of the compounds listed are described below. The general reference was to Journal of the Chemical Society, Perkin I 1977, pages 2047 to 2052, U.S. Patent No. 3,725,067 and Unexamined Published Japanese Patent Application Nos. 99437/1984 and 42045/1983.

### Synthesis of compound (1):

The reaction scheme is shown below:

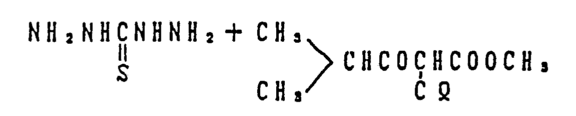

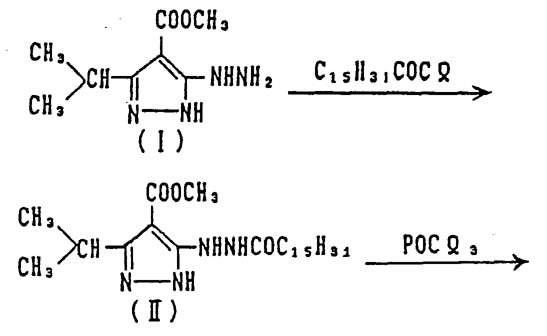

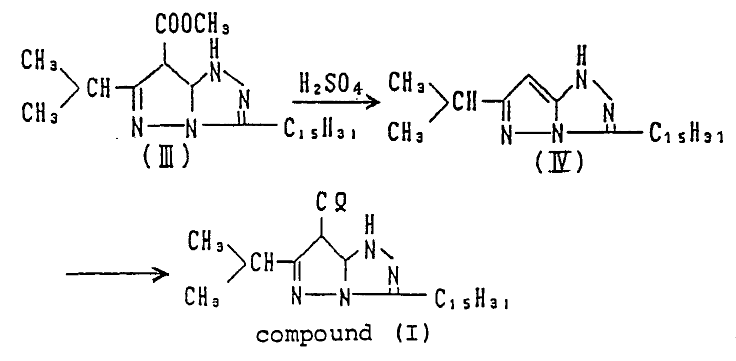

### (1) Synthesis of Compound (I):

Thiocarbohydrazide (53 g) was suspended in alcohol, followed by the addition of benzaldehyde (53 g) under boiling and agitation. After 10 minutes, a-chloroisobutyrylmethylacetate (89.5 g) was added dropwise, and after another 10 minutes, hydrazinehydrate was added dropwise for 1 hour, followed then by boiling and agitation for another one hour. Thereafter, insoluble substances were removed and the alcohol was distilled off. The residue was crystallized by addition of 250 ml of toluene and recovered by filtration. The recovered crystals were washed well with water, then dried and thereafter recrystallized from the toluene to obtain the end product.

### (2) Synthesis of Compound (II):

Compound (1), 27 g, was added to a mixed solution of 250 ml of acetonitrile and 17 g of triethylamine, followed by the dropwise addition of 37 g of parmitoyl chloride for 30 minutes under agitation and refluxing. The mixture was thereafter further refluxed for 2 hours. The reaction mixture was cooled and the resulting crystals were recovered by filtration. The recovered crystals were washed well with water and dried to obtain the end product almost quantitatively.

### (3) Synthesis of Compound (III):

Compound (II), 52 g, and 20 g of phosphorus oxychloride were added in to 500 ml of toluene, followed by refluxing for 2 hours. Thereafter, the toluene was distilled off under reduced pressure. To the residue were added 300 ml of acetonitrile and 25.6 g of pyridine, and the mixture was boiled for 2 hours. The acetonitrile was distilled off from the reaction mixture, to which water was added to form crystals. The resulting crystals were recovered by filtration, washed with water, dried and thereafter recrystallized from the acetonitrile to obtain the end product (III).

### (4) Synthesis of Compound (IV):

Compound (III), 20 g, was added to a mixture of 100 ml of glacial acetic acid and 10 ml of concentrated sulfuric acid. The resulting mixture was refluxed for 15 hours and left to cool. Into the reaction mixture was then poured an aqueous caustic soda solution comprising 100 ml of water and 18 g of caustic soda under agitation. After cooling, the deposited crystals were recovered by filtration and washed fully with water. After drying, the recovered crystals were purified by column chromatography on silica gel using benzene-acetone as a solvent.

### (5) Synthesis of Compound (I):

Twenty grams of compound (IV) was dissolved in chloroform and, to the solution, an equivalent amount of N-chlorosuccinimide was added. The mixture was held at 20°C for one hour to perform the reaction. The reaction mixture was then washed with dilute alkali to remove the resulting succinimide. The chloroform was distilled off and the residue was purified by column chromatography on silica gel using benzene-acetone as a solvent. The purified residue was identified as the end compound or compound (1) by NMR spectrum.

As for other compounds, the 1H-pyrazolo[3,2-c]-S-triazole nucleus free of the leaving group X can be synthesized by the method used in the production of compound (1).

Compounds (3), (12) and (18) can be synthesized by referring to the general method described in U.S. Patent No. 3,725,067. Compounds (4) and (19) can be brominated by, for example, the method described in Example 31 of said U.S. Patent and the resulting bromine-substituted product reacted with a silver salt of methanesulfonic acid. Compounds (9), (20) and (22) can also be synthesized in accordance with the method of synthesis described in said U.S. Patent. Compounds (6), (13), (15) and (17) can be synthesized by referring to the general method of synthesis described in Unexamined Published Japanese Patent Application No. 99437/1984.

The silver halide color photographic material of the present invention may contain conventional dye- forming couplers.

Known open-chain ketomethylene couplers may be used as yellow-forming couplers. Benzoylacetanilide and pivaloylacetanilide compounds are particularly useful. Specific examples of the usable yellow forming couplers are described in U.S. Patent Nos. 2,875,057,3,265,506,3,408,194,3,551,155, 3,582,322, 3,725,072, and 3,891,445; German Patent No. 1,547,868, German Patent Application (OLS) Nos. 2,219,917, 2,261,361 and 2,414,006; British Patent No. 1,425,020; Japanese Patent Publication No. 10783/1976, Unexamined Published Japanese Patent Application Nos. 26133/1972, 73147/1983, 102036/ 1976, 6341/1975, 123342/1975, 130442/1975, 21827/1976, 87650/1975, 82424/1977 and 115219/1977.

Usable cyan forming couplers are phenolic and naptholic compounds. Specific examples are found in U.S. Patent Nos. 2,369,929, 2,434,272, 2,474,293, 2,521,908, 2,895,826, 3,034,892, 3,311,476, 3,458,315, 3,476,563, 3,583,97, 3,591,383, 3,767,411 and 4,004,929; German Patent Application (OLS) Nos. 2,414,830 and 2,454,329; and Unexamined Published Japanese Patent Application Nos. 59838/1973, 26034/1976, 5055/1973, 146828/1976, 69624/1977 and 90932/1977.

As magenta forming couplers, one or more of the couplers prepared in accordance with the present invention may be used. They may also be used in combination with known magenta couplers such as pyrazolone compounds, indazolone compounds, cyanoacetyl compounds, pyrazolinobenzimidazole compounds and pyrazolotriazole compounds. It should however be emphasized that at least one of the magenta couplers incorporated in the silver halide color photographic material of the present invention must be the coupler defined in accordance with the invention.

The coupler of the present invention may also be used in combination with colored couplers capable of color correction, or development inhibitor releasing couplers (DIR couplers) that are effective for producing improved image quality.

The magenta coupler of the present invention and the respective couplers associated thereto may be introduced into silver halide emulsion layers by any known method such as one described in U.S. Patent No. 2,322,027. For example, the couplers are dispersed in hydrophilic colloids after being dissolved in high-boiling organic solvents or low-boiling organic solvents. Examples of the former type include alkyl esters of phthalic acid (e.g. dibutyl phthalate and dioctyl phthalate), phosphate esters (e.g. diphenyl phosphate, triphenyl phosphate, tricresyl phosphate and dioctylbutyl phosphate), citrate esters (e.g. tributyl acetylcitrate), benzoate esters (e.g. octyl benzoate), alkylamides (e.g. diethyl laurylamide), aliphatic acid esters (e.g. dibutoxyethyl succinate and dioctyl azelate) and trimesic acid esters (e.g. tributyl trimesate). The low-boiling organic solvents are those which boil at 30°C to 150°C, and examples are lower alkyl acetates (e.g. ethyl acetate and butyl acetate), ethyl propionate, secondary butyl alcohol, methyl isobutyl ketone, 6-ethoxyethyt acetate and methyl cellosolve acetate. The high-boiling organic solvents may be used in combination with the low-boiling organic solvents.

Dispersion methods using polymers may also be used and such methods are described in Japanese Patent Pubication No. 39853/1976 and Unexamined Published Japanese Patent Application No. 59943/1976.

The magenta coupler of the present invention is incorporated in a silver halide emulsion layer usually in an amount of from 0.005 to 2 moles, preferably from 0.03 to 0.5 mole, per mole of silver halide.

The magenta coupler of the present invention forms a satisfactorily light-fast dye image, but even higher light fastness may be obtained by using an anti-fading agent or by overlaying the emulsion layer of interest with a layer containing an ultraviolet absorber.

Illustrative anti-fading agents include hydroquinone derivatives of the type described in U.S. Patent Nos. 2,360,290, 2,418,613, 2,673,314, 2,701,197, 2,704,713, 2,728,659, 2,732,300, 2,735,765, 2,710,801 and 2,816,028, as well as British Patent No. 1,363,291; gallic acid derivatives as described in U.S. Patent Nos. 3,457,079 and 3,069,262; p-alkoxyphenols of the type described in U.S. Patent Nos. 2,735,765 and 3,698,909, as well as Japanese Patent Publication No. 20977/1974 and 6623/1977; p-oxyphenol derivatives of the type described in U.S. Patent Nos. 3,432,300, 3,573,050, 3,574,627 and 3,764,337, as well as Unexamined Published Japanese Patent Application Nos. 35633/1977,147434/1977 and 152225/1977; and bisphenols as described in U.S. Patent No. 3,700,455.

Exemplary ultraviolet absorbers includes aryl-substituted benzotriazole compounds (as described in U.S. Patent No. 3,533,794), 4-thiazolidone compounds (as described in U.S. Patent Nos. 3,314,794 and 3,352,681), benzophenone compounds (as described in Unexamined Published Japanese Patent Application No. 2784/1971), cinnamic acid ester compounds (as described in U.S. Patent Nos. 3,705,805 and 3,707,375), butadiene compounds (as described in U.S. Patent No. 4,045,229) and benzoxidole compounds (as described in U.S. Patent No. 3,700,455). Other compounds usable as UV absorbers are found in U.S. Patent No. 3,499,762 and Unexamined Published Japanese Patent Application No. 48535/1979.

Any of the silver halides that are incorporated in conventional silver halide emulsions may be used in the present invention and they include silver bromide, silver chloride, silver iodobromide, silver chlorobromide and silver chloroiodobromide. In order to provide sensitivity for the desired spectral wavelength region, the silver halides used in the present invention may be spectrally sensitized by suitably selected sensitizing dyes. Usable dyes include cyanine, merocyanine, complex cyanine, complex merocyanine, holopolar cyanine, hemicyanine, styryl and hemioxonole dyes.

Useful sensitizing dyes are described in, for example, German Patent No. 929,080, U.S. Patent Nos. 2,231,658, 2,493,748, 2,503,776, 2,519,001, 2,912,329, 3,656,959, 3.672.897. 3,694,217, 4,025,349 and 4,046,572; British Patent No. 1,242,588; and Japanese Patent Publication Nos. 14030/1969 and 24844/1977.

These sensitizing dyes may be used either individually or in combination. Combined sensitizing dyes are often used for the purpose of supersensitization, as typically described in U.S. Patent Nos. 2,688,545, 2,977,229, 3,397,060, 3,522,052, 3,527,641, 3,617,293, 3,628,964, 3,666,480, 3,672,898, 3,679,428, 3,703,377, 3,769,301,3,814,609,3,837,862 and 4,026,707; British Patent Nos. 1,344,281 and 1,507,803; Japanese Patent Publication Nos. 4936/1968 and 12375/1978; and Unexamined Published Japanese Patent Application Nos. 110618/1977 and 109925/1977.

The silver halide emulsion used in the present invention may incorporate a variety of known photographic additives such as those described in Research Disclosure No. 17643.

The silver halide color photographic material of the present invention may use any support material that is properly selected from among known materials depending on the intended use such as plastic films, plastic laminated paper, baryta paper and synthetic paper.

The silver halide color photographic material of the invention may adopt any of the layer arrangements commonly used in the photographic industry.

The silver halide color photographic material of the invention is exposed and thereafter subjected to color development by a variety of photographic processing techniques. The color developer used to process this photographic material may contain any of the known aromatic primary amine color developing agents that are extensively used in various color photographic processes. Such developing agents include aminophenolic and p-phenylenediamine derivatives. These compounds are generally used in salt forms, such as hydrochlorides or sulfates, which are stabler than the free state. These compounds are used in concentrations that generally range from 0.1 to 30 g, preferably from 1 g to 1.5 g, per liter of the color developer.

Illustrative aminophenolic developing agents include o-aminophenol, p-aminophenol, 5-amino-2- oxytoluene, 2-amino-3-oxytoluene, and 2-oxy-3-amino-1,4-dimethylbenzene.

Particularly useful primary aromatic amino color developing agents are N,N'-dialkyl-p-phenylenediamine compounds wherein the alkyl or phenyl group may have a suitable substituent. Among these compounds, the following are particularly advantageous: N.N'-diethyl-p-phenylenediamine hydrochloride, N-methyl-p-phenylenediamine hydrochloride, N,N'-dimethyl-p-phenylenediamine hydrochloride, 2-amino-5-(n-ethyl-N-dodecylamino)-toluene, N-ethyl-N-(3-methanesulfonamidoethyl-3-methyl-4-aminoaniline sulfate, N-ethyl-N-¡3-hydroxyethylaminoaniline, 4-amino-3-methyl-N,N'-diethylaniline, and 4-amino-N-(2-methoxyethyl)-N-ethyl-3-methylaniline-p-toluene sulfonate.

In addition to these primary aromatic amino color developing agents, the color developer used in the processing of the photographic material of the present invention may contain a variety of additives that are commonly incorporated in color developers and such additives include alkali agents (e.g. sodium hydroxide, sodium carbonate and potassium carbonate), alkali metal sulfites, alkali metal bisulfites, alkali metal thiocyanates, alkali metal halides, benzyl alcohol, water softeners and thickeners. The pH of the color developer is usually at least 7 and most generally ranges from 10 to 13.

After color development, the photographic material of the present invention is usually processed by a solution having the fixing ability. If this solution is a fixing bath, its use is preceded by a bleaching step. The bleaching bath used in the bleaching step or the bleaching agent used in a bleach-fixing bath is generally made of a metal complex salt of an organic acid. This metal complex salt has the ability not only to oxidize metallic silver (i.e. formed as a result of development) into silver halide but also to ensure complete color formation by a color former. The structure of this metal complex salt is such that an organic acid such as an aminopolycarboxylic acid, oxalic acid or citric acid is coordinated to a metal ion such as iron, cobalt-or copper. The organic acids most preferred for use in forming metal complex salts are polycarboxylic acids or aminopolycarboxylic acids. The polycarboxylic acids or aminopolycarboxylic acids may be in the form of alkali metal salts, ammonium salts or water-soluble salts.

Typical examples of polycarboxylic acids or aminopolycarboxylic acids are listed below:
(1) ethylenediaminetetraacetic acid;
(2) diethylenetriaminepentaacetic acid;
(3) ethylenediamine-N-(β-oxyethyl)-N,N',N'-triacetic acid;
(4) propylenediaminetetraacetic acid;
(5) nitrilotriacetic acid;
(6) cyclohexanediaminetetraacetic acid;
(7) iminodiacetic acid;
(8) dihydroxyethylglycincitric acid (or tartaric acid);
(9) ethyletherdiaminetetraacetic acid;
(10) glycoletherdiaminetetraacetic acid;
(11) ethylenediaminetetrapropionic acid;
(12) phenylenediaminetetraacetic acid;
(13) ethylenediaminetetraacetic acid disodium salt;
(14) ethylenediaminetetraacetic acid tetra(trimethylammonium) salt;
(15) ethylenediaminetetraacetic acid tetrasodium salt;
(16) diethylenetriaminepentaacetic acid pentasodium salt;
(17) ethylenediamine-N-(¡3-oxyethyl)-N,N',N'-triacetic acid sodium salt;
(18) propylenediaminetetraacetic acid sodium salt;
(19) nitrilotriacetic acid sodium salt; and
(20) cyclohexanediaminetetraacetic acid sodium salt.

In addition to metal complex salts of these organic acids which are used as bleaching agents, the bleaching bath used in processing the color photographic material of the present invention may contain a variety of additives, and preferred additives are rehalogenating agents such as alkali or ammonium halides (e.g. potassium bromide, sodium bromide, sodium chloride and ammonium bromide), metal salts and chelating agents. Any other additives that are conventionally incorporated in bleaching baths may also be used and they include pH buffers (e.g. borate, oxalate, acetate, carbonate and phosphate salts), alkylamines and polyethylene oxides.

The fixing bath and bleach-fixing bath may also contain one or more pH buffers that are selected from among, say, sulfites (e.g. ammnium sulfite, potassium sulfite, ammonium bisulfite, potassium bisulfite, sodium bisulfite, ammonium metabisulfite, potassium metabisulfite, and sodium metabisulfite), and a variety of acids or salts (e.g. boric acid, borax, sodium hydroxide, potassium hydroxide, sodium carbonate, potassium carbonate, sodium bicarbonate, potassium bicarbonate, acetic acid, sodium acetate and ammonium hydroxide).

If the photographic material of the present invention is processed in a bleach-fixing bath as it is supplied with a blix replenisher, thiosulfates, thiocyanates, sulfites or other salts may be incorporated either in the bleach-fixing bath or in the replenisher that is fed to said blix bath.

In order to increase the activity of the bleach-fixing bath used in processing the photographic material of the present invention, air or oxygen may be blown into the tank containing the bleach-fixing bath or its replenisher. Alternatively, a suitable oxidant such as hydrogen peroxide, bromate or persulfate may be added into the tank.

The following examples are provided for further illustration of the claimed photographic material but are not to be construed as limiting the invention.

### Example 1

One tenth of a mole, per mole of silver, of one of the magenta couplers listed in Table 1 (which follow) was mixed with an equal weight of tricresyl phosphate and three times the coupler's weight of ethyl acetate, and the mixture was heated to 60°C to form a complete solution. The solution was then mixed with 1,200 ml of 5% aqueous gelatin solution containing 120 ml of a 5% aqueous solution of Alkanol B (trade name of du Pont for alkylnaphthalene sulfonate). The mixture was emulsified with an ultrasonic disperser and the dispersion obtained was added to 4 kg of a green-sensitive silver iodobromide emulsion (containing 6 mol% Agl). To the mixture, 120 ml of a 2% solution (water:methanol = 1:1) of 1,2-bis(vinylsulfonyl)-ethane was added as a hardener, and the so prepared coating solution was applied to a subbed transparent polyester base, and the web was dried to provide a sample of color photographic material (with silver deposit of 20 mg/100 cm²). The other samples were prepared by the same procedure.

Each of the samples thus prepared was subjected to exposure through an optical wedge as in the conventional process and subsequently processed by the following scheme. The results of such photographic processing are shown in Table 1 below.

### Processing scheme

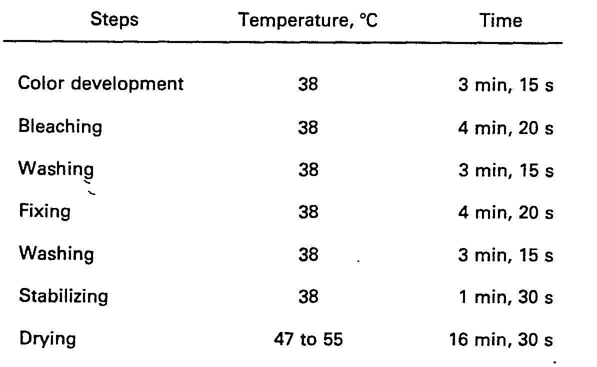

The formulation of each of the processing solutions used is indicated below.

### Color developer

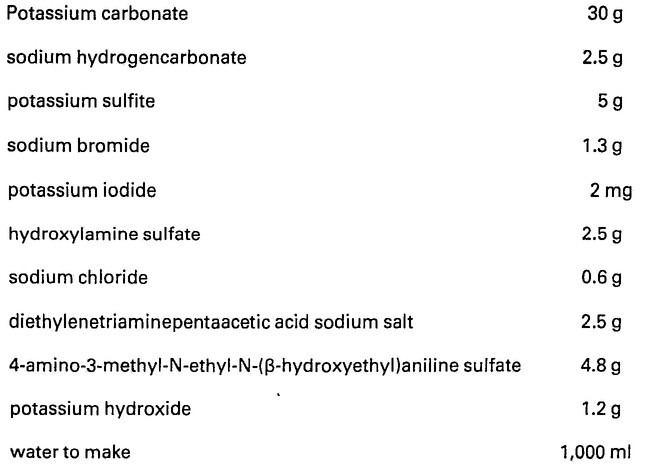

pH adjusted to 10.06 by addition of potassium hydroxide or 20% H₂SO₄.

### Bleaching bath

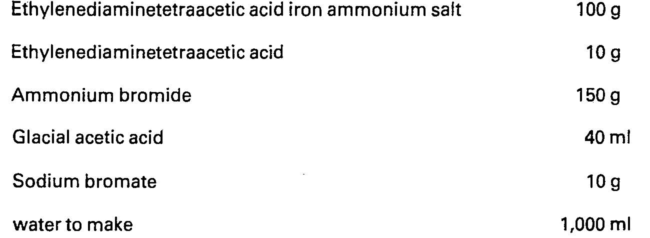

pH adjusted to 3.5 by addition of ammonia water or glacial acetic acid.

### Fixing Bath:

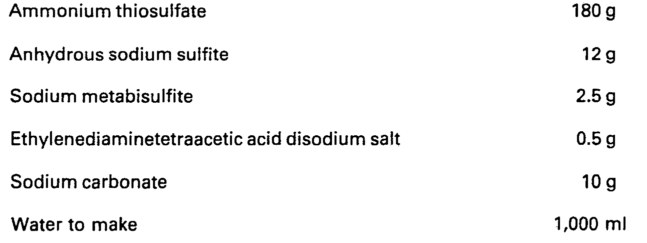

### Stabilizing bath

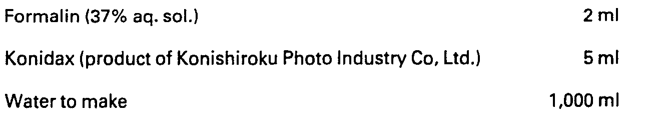

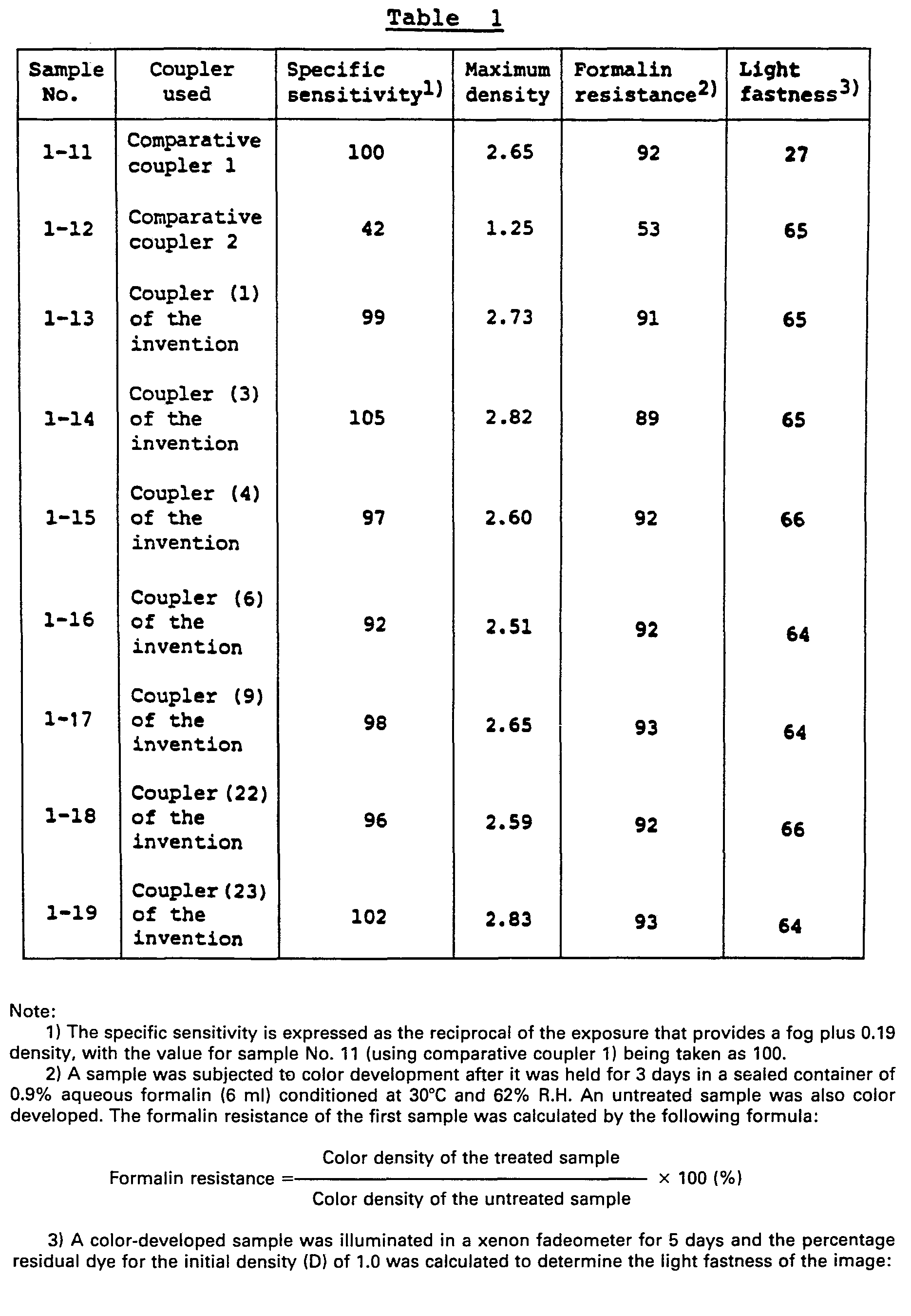

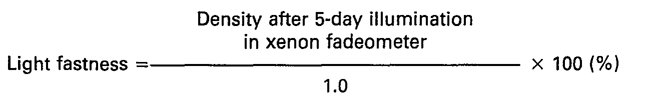

### Comparative coupler 1

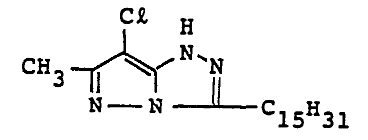

### Comparative coupler 2

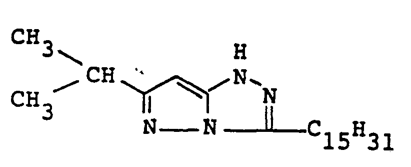

The data in Table 1 show that the couplers prepared in accordance with the present invention satisfied all the requirements for high color density and the production of formalin-resistant and light-fast dye images.

### Example 2

Sample Nos. 1-11 to 1-19 prepared in Example 1 were exposed through an optical wedge and subsequently processed by the following scheme. The result are shown in Table 2. The specific sensitivity and light fastness were measured by the same methods as used in Example 1.

### Processing scheme:

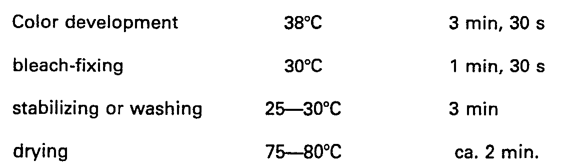

The solutions used in this scheme had the following formulations.

### Color developer

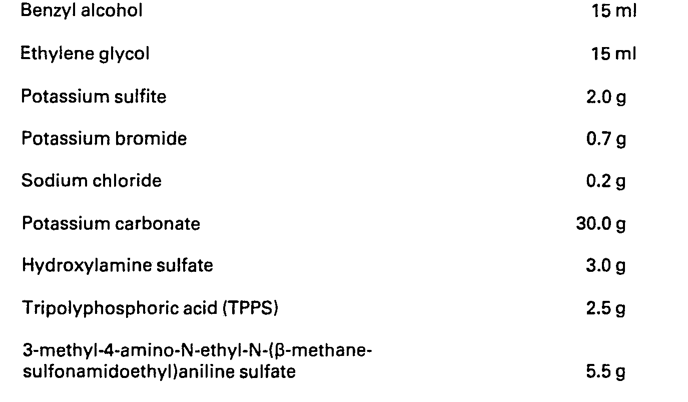

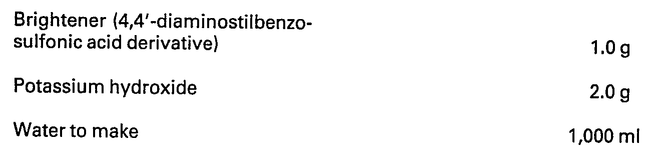

ph adjusted to 10.20

### Bleach-fixing bath

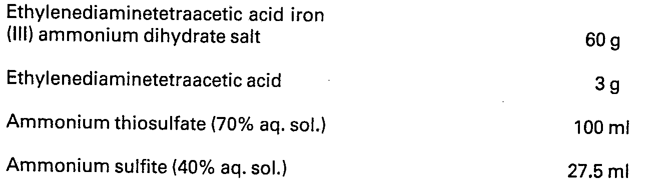

pH adjusted to 7.1 by addition of potassium carbonate or glacial acetic acid

### Stabilizing bath

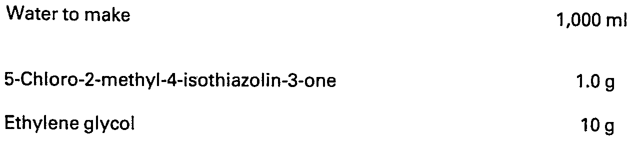

### Ethylene glycol

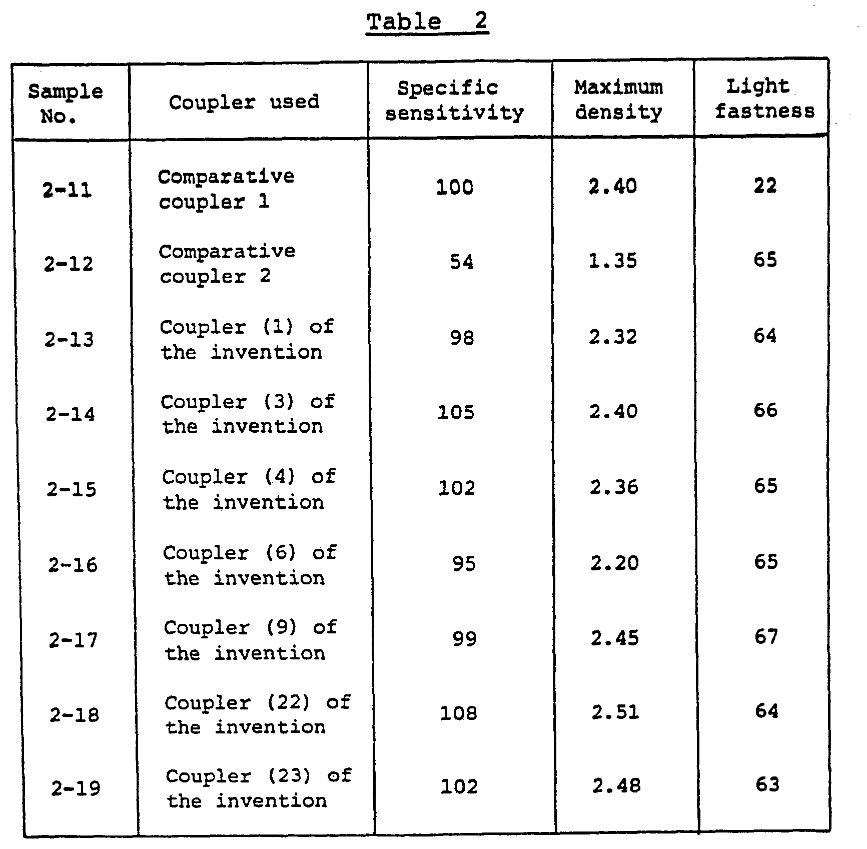

As the data in Table 2 show, the samples containing the magenta couplers prepared in accordance with the present invention were superiorto those containing the comparative couplers in respect to sensitivity, color density and the production of light-fast dye images.

### Example 3

A sample of silver halide color photographic material was prepared by coating the following layers in sequence on a support made of polyethylene coated paper containing anatase type Ti0₂. The amounts of the additives incorporated in each of the layers described below are based on an area of 100 cm^{2.}
(1) Layer containing 20 mg of gelatin, 5 mg in terms of silver of a blue-sensitive silver chlorobromide emulsion, and 3 mg of dioctyl phthalate coupler solvent having dissolved therein 8 mg of Y-coupler^{*} and 0.1 mg of 2,5-di-t-octylhydroquinone:
(2) Interlayer containing 12 mg of gelatin, and 2 mg of dibutyl phthalate UV absorber solvent having 0.5 mg of 2,5-di-t-octylhydroquinone and 4 mg of UV absorber^{*} dissolved therein:
(3) Layer containing 18 mg of gelatin, 4 mg in terms of silver of a green-sensitive silver chlorobromide emulsion, and 2.5 g of dioctyl phthalate coupler solvent having dissolved therein 5 mg of M-coupler^{*}, 2 mg of antioxidant^{*} and 0.2 mg of 2,5-di-t-octylhydroquinone:
(4) Interlay having the same composition as (2):
(5) Layer containing 16 mg of gelatin, 4 mg in terms of silver of a red-sensitive silver chlorobromide emulsion, and 2.0 mg of tricresyl phosphate coupler solvent having dissolved therein 3.5 mg of C-coupler^{*} and 0.1 mg of 2,5-di-t-octylhydroquinone:
(6) Gelatin protective layer containing 9 mg of gelatin.

Each of the layers (1) to (6) also contained a coating aid, while layers (4) and (6) further contained a gelatin crosslinking agent. The ultraviolet absorber used in each of the layers (2) and (4) was a mixture of UV-1 and UV-2 having the structures shown below. The antioxidant incorporated in layer (3) was di-t-pentylhydroquinone-di-octyl ether.

Thirteen samples of multi-layered photographic material were prepared as above and each was processed as in Example 2. The specific types of the Y-coupler, M-coupler and C-coupler used, and the results of the photographic processing are shown in Table 3 below. Each of the samples was checked for its magenta density after exposure to white light. The specific sensitivity and light fastness were measured by the same methods as used in Example 1.

The data in Table 3 show the improved light fastness of the dye images produced by using the magenta couplers prepared in accordance with the present invention. It was also clear that the light fastness of the images could be further improved by using UV absorbers in combination with the magenta couplers.

### ^{*}Ultraviolet absorber

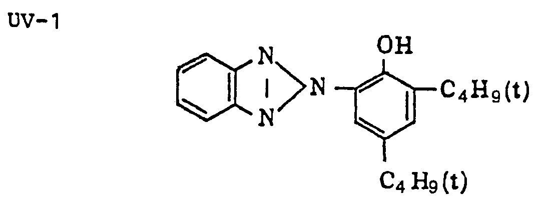

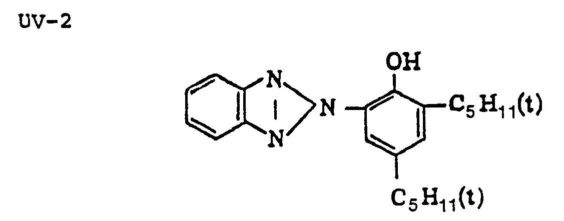

### ^{*}Y-couplers

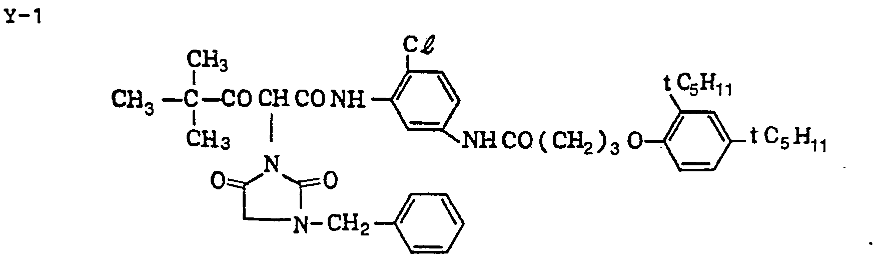

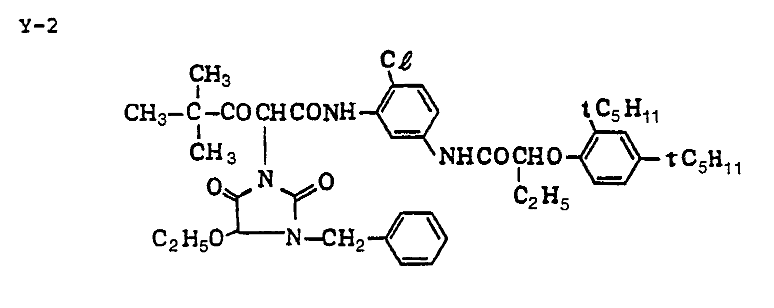

### ^{*}C-couplers

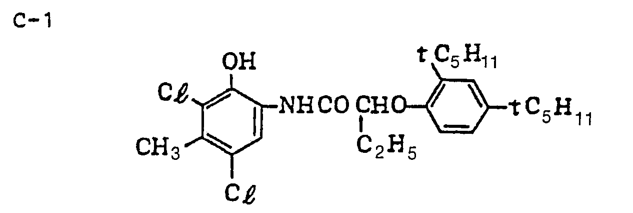

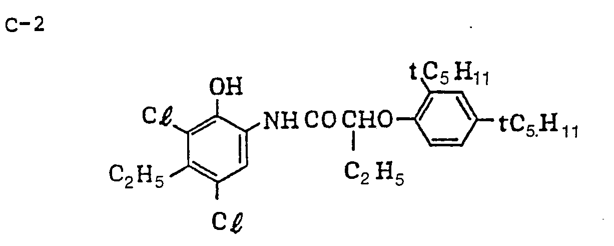

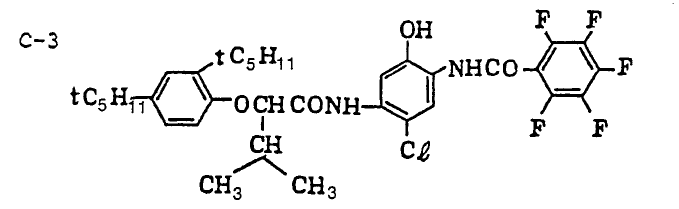

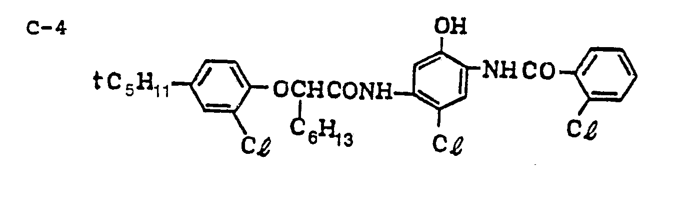

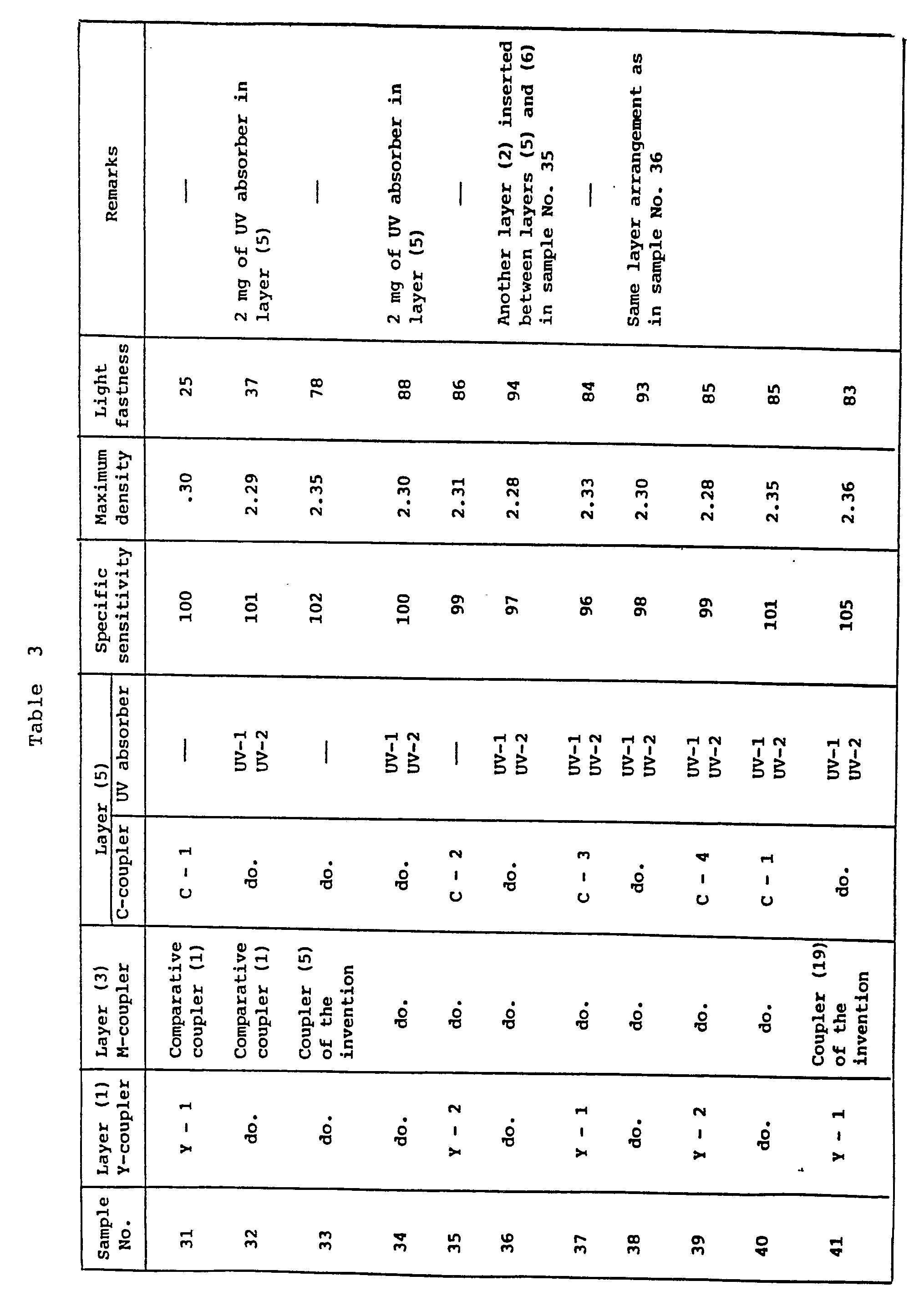

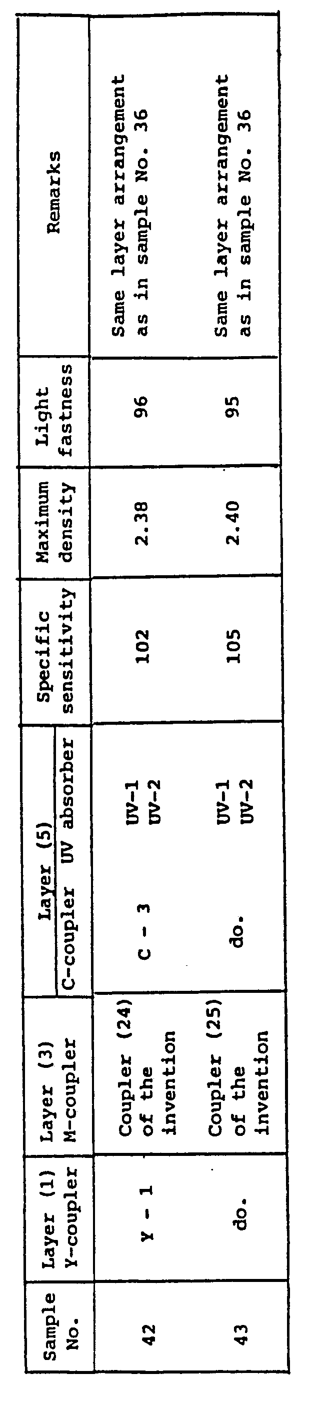

## Claims

1. A silver halide color photographic material comprising a support and at least one silver halide emulsion layer containing at least one 1 H-pyrazolo[3,2-c]-S-triazole type magenta coupler substituted at 6- position by an optionally substituted secondary alkyl group and at 7-position by a leaving group other than a hydrogen atom susceptible to displacement during reaction of the compound with the oxidation product of a color developing agent other than the compounds of the formulae: 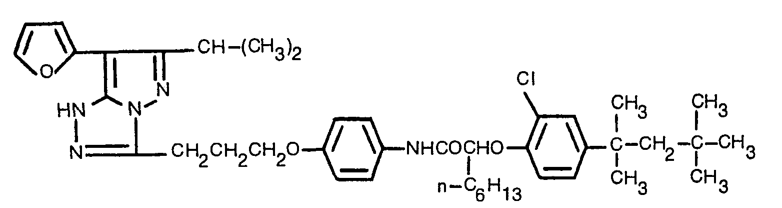 and

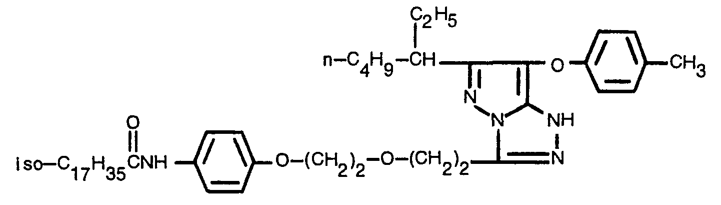

2. A silver halide color photographic material according to claim 1, wherein the magenta coupler is a compound of the formula (I): 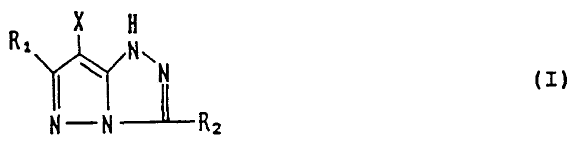 where R₁ is an optionally substituted secondary alkyl group; R₂ is an optionally substituted alkyl, aryl or heterocyclic group or a group bonded to the carbon atom of the nucleus through a bonding group selected from among an oxygen atom, a nitrogen atom, a sulfur atom, acylamino, carbamoyl, sufonamido, sufamolycarbonyl, carbonyloxy, oxycarbonyl, ureido, thioureido, thioamido, sulfone and sulfonyloxy groups; and X is a leaving group other than a hydrogen atom susceptible to displacement during reaction of the compound of formula (I) with the oxidation product of a color developing agent.

3. A silver halide color photographic material according to claim 1 or claim 2, wherein the magenta coupler is a compound of formula (II): 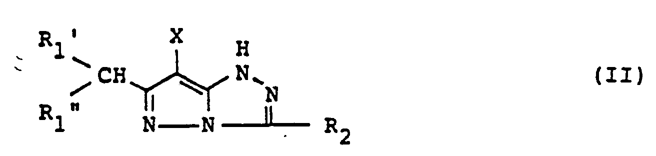 wherein R₁' and R₁'' are the same or different and each is a halogen atom, a cyano group, a nitro group or a group R₂; and R₂ and X are groups as defined in claim 2 with respect to formula (1).

4. A silver halide color photographic material according to claim 2, wherein R, is a group

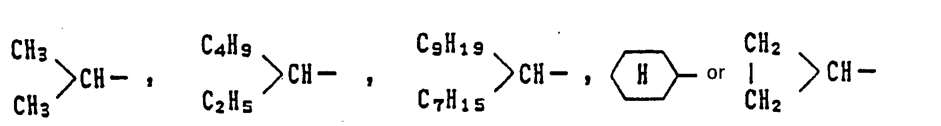

5. A silver halide color photographic material according to any one of claims 2 to 4, wherein R₂ is a group of formula (III), (IV) or (V): 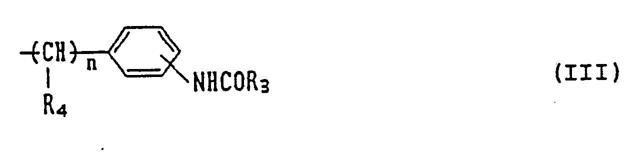 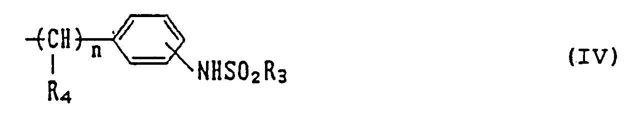 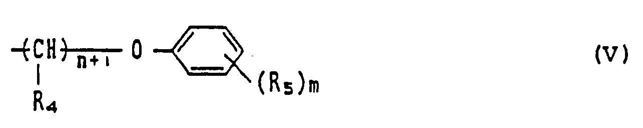 wherein R₃ is an alkyl, aryl or heterocyclic group; R₄ is a hydrogen atom or an alkyl group; R₅ is an alkyl, alkoxy, sulfamoyl, sulfonamido or sulfonyl group; m is an integer of 1 to 3; and n is 0 or an integer of 1 to 4.

6. A 1 H-pyrazolo[3,2-c]-S-triazole type magenta coupler as defined in any one of claims 1 to with the disclaimer of claim 1.

## Patentansprüche

1. Farbphotographisches Silberhalogenid-Aufzeichnungsmaterial aus einem Schichtträger und mindestens einer Silberhalogenidemulsionsschicht mit mindestens einem in der 6-Stellung durch eine gegebenenfalls substituierte sek.-Alklyl-gruppe und in 7-Stellung durch eine von einem Wasserstoffatom verschiedene, bei der Umsetzung der betreffenden Verbindung mit dem Oxidationsprodukt eines Farbentwicklers abspaltbare Gruppe substituierten Purpurrotkuppler vom Typ 1H-Pyrazolo [3,2-c]-S-triazole, wobei jedoch folgende Verbindung ausgenommen sind: 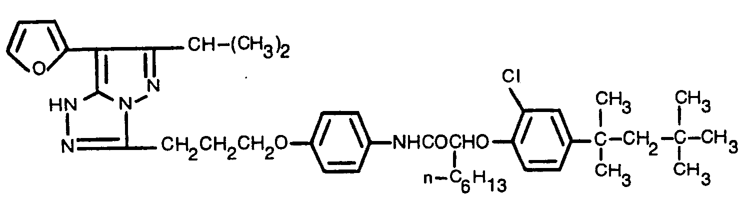 und

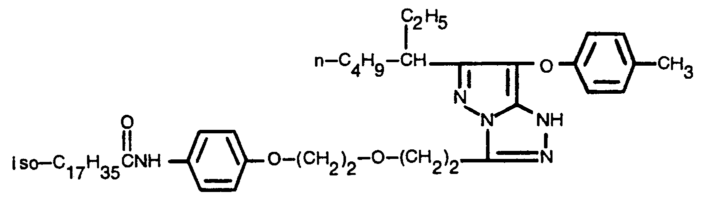

2. Farbphotographisches Silberhalogenid-Aufzeichnungsmaterial nach Anspruch 1, dadurch gekennzeichnet, daß es sich bei dem Purpurrotkuppler um eine Verbindung der Formel (I) 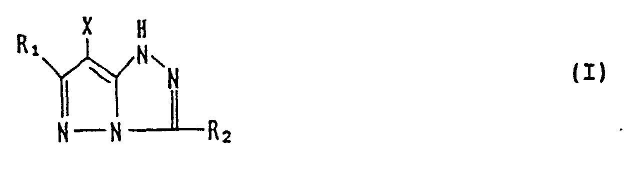 worin bedeuten: R, eine gegebenenfalls substituierte sek.-Alkylgruppe; R₂ eine gegenbenenfalls substituierte Alkylgruppe, Arylgruppe oder heterocyclische Gruppe oder eine Gruppe, die an das Kernkohlenstoffatom über eine verbindende Gruppe, ausgewählt aus einem Sauerstoff-, Stickstoff-, oder Schwefelatom oder einer Acylamino-, Carbamoyl-, Sulfonamido-, Sulfamoylcarbonyl-, Carbonyloxy-, Oxycarbonyl-, Ureido-, Thioureido-, Thioamido-, Sulfon- oder Sulfonyloxygruppe, und X eine von einem Wasserstoffatom verschiedene, bei der Umsetzung der betreffenden Verbindung der Formel (I) mit dem Oxidationsprodukt eines Farbentwicklers abspaltbare Gruppe handelt.

3. Farbphotographisches Silberhalogenid-Aufzeichnungsmaterial nach Anspruch 1 oder 2, dadurch gekennzeichnet, daß es sich bei dem Purpurrotkuppler um eine Verbindung der Formel (II) 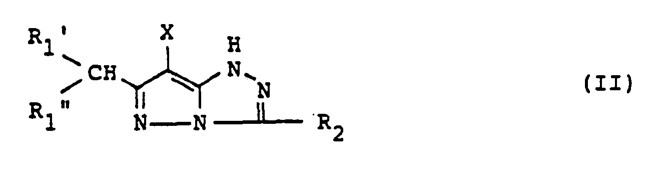 worin R₁' und R,", die gleich oder verschieden sind, jeweils für ein Halogenatom eine Cyanogruppe, eine Nitrogruppeodder eine Gruppe R₂ stehen und R₂ und X die in Anspruch 2 im Zusammenhang mit Formel (I) angegebene Bedeutung besitzen, handelt.

4. Farbphotographisches Silberhalogenid-Aufzeichnungsmaterial nach Anspruch 2, dadurch gekennzeichnet, daß R₁ für eine Gruppe

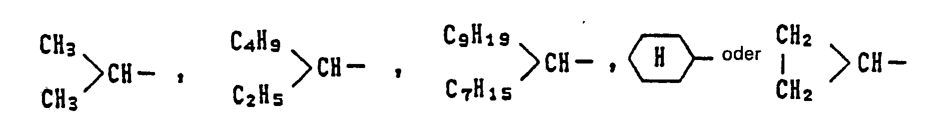

5. Farbphotographisches Silberhalogenid-Aufzeichnungsmaterial nach einem der Ansprüche 2 bis 4, dadurch gekennzeichnet, daß R₂ für eine Gruppe der Formeln (III), (IV) oder (V) 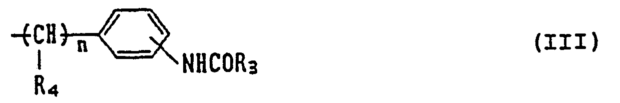 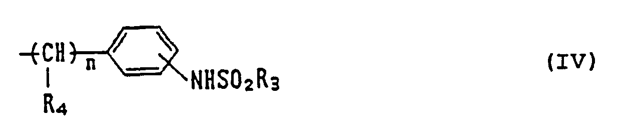 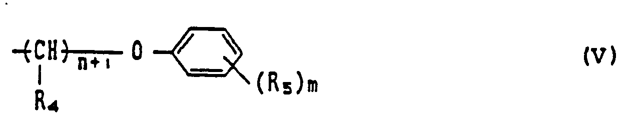 mit R₃ gleich einer Alkylgruppe, Arylgruppe oder heterocyclischen Gruppe, R₄ gleich einem Wasserstoffatom oder einer Alkylgruppe, R₅ gleich einer Alkyl-, Alkoxy-, Sulfamoyl-, Sulfonamido- oder Sulfonylgruppe, m gleich einer ganzen Zahl von 1 bis 3 und n gleich 0 oder einer ganzen Zahl von 1 bis 4 steht.

6. 1H-Pyrazolo[3,2-c]-S-triazolartiger Purpurrotkuppler nach einem der Ansprüche 1 bis 5 unter Berücksichtigung des Disclaimers von Anspruch 1.

## Revendications

1. Matériau photographique couleur à base d'halogénure d'argent, comprenant un support et au moins une couche d'émulsion d'halogénure d'argent contenant au moins un coupleur magenta du type 1 H-^{p}yrazolo[3,2-c]-S-triazole substitue en position 6 par un groupe alkyle secondaire éventuellement substitué et en position 7 par un groupe partant autre qu'un atome d'hydrogène, susceptible de se déplacer pendant la réaction du compose avec le produit d'oxydation d'un agent de developpement couleur autre que les composes de formules: 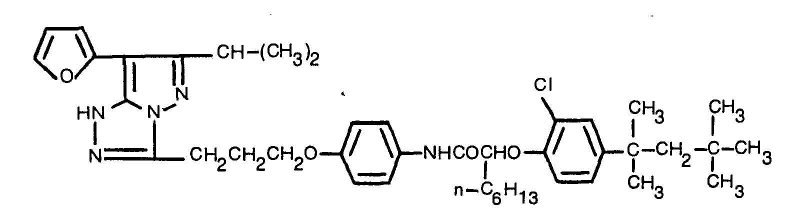 et

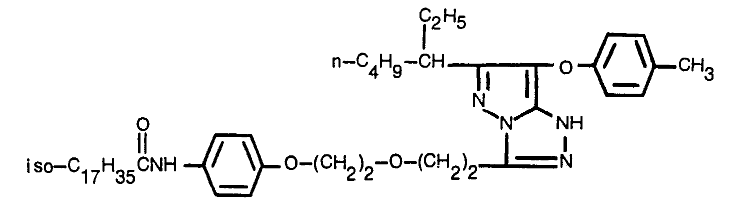

2. Matériau photographique couleur à base d'halogénure d'argent selon la revendication 1, dans lequel le coupleur magenta est un compose de formule (I): 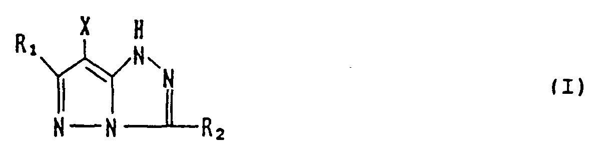 où R, est un groupe alkyle secondaire éventuellement substitué, R₂ est un groupe alkyle, aryle ou hétérocyclique eventuellement substitué ou un groupe lié à l'atome de carbone du noyau par l'intermédiaire d'une groupe de liaison choisi parmi un atome d'oxygène, un atome d'azote, un atome de soufre, un groupe acylamino, carbamoyle, sulfonamido, sulfamoylcarbonyle, carbonyloxy, oxycarbonyle, ureido, thioureido, thioamido, sulfone et sulfonyloxy; et X est un groupe partant autre qu'un atome d'hdrogène, susceptible de se déplacer pendant la réaction du composé de formule (I) avec le produit d'oxydation d'un agent de developpement couleur.

3. Matériau photographique couleur à base d'halogénure d'argent selon la revendication 1 ou 2, dans lequel le coupleur magenta est un composé de formule (II): 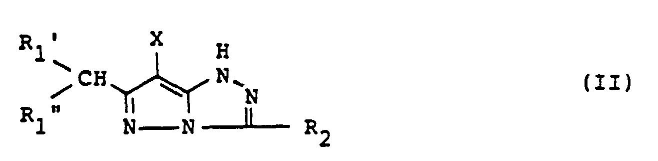 où R₁' et R₁'' sont identiques ou différents, et chacun représente un atome d'halogene, un groupe cyano, un groupe nitro ou un groupe R₂, et R₂ et X sont des groupes tel que définis dans la revendication 2 concernant la formule (1).

4. Matériau photographique couleur à base d'halogénure d'argent selon la revendication 2, dans lequel R₁ est un groupe

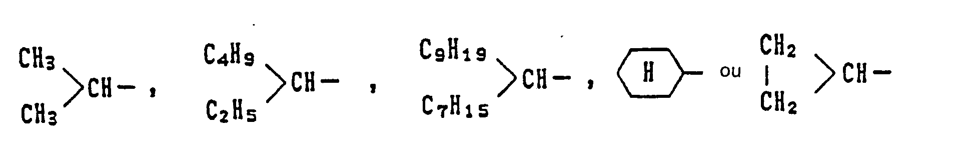

5. Matériau photographique couleur à base d'halogénure d'argent selon l'une quelconque des revendications 2 à 4, dans lequel R₂ est un groupe de formule (III), (IV) ou (V): 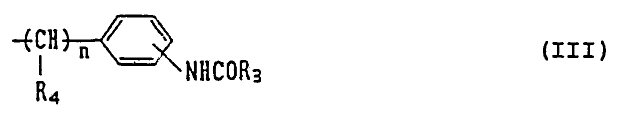 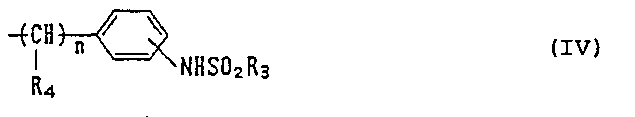 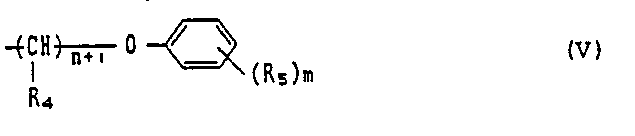 où R₃ est un groupe alkyle, aryle ou hétérocyclique; R₄ est un atome d'hydrogène ou un groupe alkyle; R₅ est un groupe alkyle, alcoxy, sulfamoyle, sulfonamido ou sulfonyle; m est un nombre entier de 1 à 3, et n est 0 ou un nombre entier de 1 à 4.

6. Coupleur magenta du type 1H-pyrazolo[3,2-c]-S-triazole tel que défini dans l'une quelconque des revendications 1 à 5, avec le desistement de la revendication 1.
